# EUROPÄISCHE PATENTSCHRIFT

(11) **EP 1 765 355 B1**
(45) Veröffentlichungstag und Bekanntmachung des Hinweises auf die Patenterteilung: **06.03.2013**
(21) Anmeldenummer: 05740045.9
(22) Anmeldetag: 18.04.2005
(51) Int. Cl.: A61K 31/538, A61P 11/06

(54) **BENZOXAZINE ZUR BEHANDLUNG VON ATEMWEGSERKRANKUNGEN**
BENZOXAZINE FOR TREATING RESPIRATORY TRACT DISEASES
BENZOXAZINES DESTINEES AU TRAITEMENT DE MALADIES DES VOIES RESPIRATOIRES

(30) Priorität: 22.04.2004 DE 102004019539
(43) Veröffentlichungstag der Anmeldung: 28.03.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: BOUYSSOU, Thierry, 88447 Warthausen (DE); KONETZKI, Ingo, 88447 Warthausen (DE); PESTEL, Sabine, 88448 Attenweiler (DE); SCHNAPP, Andreas, 88400 Biberach (DE); HOENKE, Christoph, 55218 Ingelheim (DE); LUSTENBERGER, Philipp, 4056 Basel (CH); RUDOLF, Klaus, 88447 Warthausen (DE); SCHROMM, Kurt, 55218 Ingelheim (DE); BUETTNER, Frank, 88448 Attenweiler (DE); HEINE, Claudia, 88400 Biberach (DE); SCHOLLENBERGER, Hermann, 55218 Ingelheim (DE)
(74) Vertreter: Simon, Elke Anna Maria
(86) Internationale Anmeldenummer: PCT/EP2005/004075
(87) Internationale Veröffentlichungsnummer: WO 2005/102350

(56) Entgegenhaltungen:
- WO-A-01/83462
- WO-A-03/000241
- WO-A-2004/045618

## Beschreibung

Die vorliegende Erfindung betrifft die Verwendung der Verbindungen der allgemeinen Formel **1** 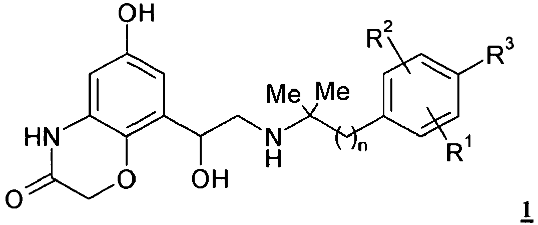 worin die Reste R¹, R² und R³ die in den Ansprüchen und in der Beschreibung genannten Bedeutungen haben können, zur Herstellung eines Arzneimittels zur Behandlung von Atemwegserkrankungen.

### Hintergrund der Erfindung

Betamimetika (β-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

Aus der WO 01/83462 und WO 03/000241 sind β-Mimetika, darunter auch solche mit einer Benzoxazin-Struktur, zur Behandlung von Atemwegserkrankungen bekannt. WO 2004/045618, die im Prioritätszeitraum der vorliegenden Anmeldung veröffentlicht wurde, offenbart die Verbindungen der Formel 1 zur Behandlung von COPD.

Zur medikamentösen Therapie von Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzü häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei. Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, daß der Patient sich relativ schnell an die regelmäßige Einnahme des Medikaments zu bestimmten Tageszeiten gewöhnen kann.

Es ist daher Aufgabe der vorliegenden Erfindung, Betamimetika bereitzustellen, die einerseits bei der Therapie von Atemwegserkrankungen einen therapeutischen Nutzen entfalten und darüber hinaus durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines zur Therapie von Asthma einmal täglich applizierbaren Arzneimittels eingesetzt werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β₂-Adrenozeptor gekennzeichnet sind.

### Detaillierte Beschreibung der Erfindung

Überraschenderweise wurde gefunden, daß die vorstehend genannten Aufgaben durch Verbindungen der allgemeinen Formel **1** gelöst werden.

Dementsprechend betrifft die vorliegende Erfindung die Verwendung einer oder mehrerer, bevorzugt einer Verbindung der allgemeinen Formel **1** 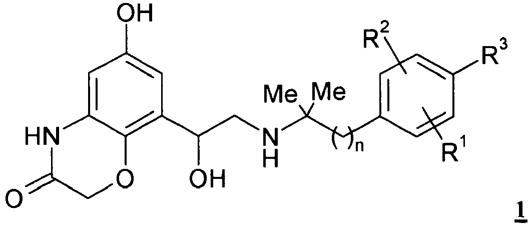 worin
- n: 1 oder 2, bevorzugt 1;
- R¹: Wasserstoff, Halogen, C₁-C₄-Alkyl oder -O-C₁-C₄-Alkyl;
- R²: Wasserstoff, Halogen, C₁-C₄-Alkyl oder -O-C₁-C₄-Alkyl;
- R³: C₁-C₄-Alkyl, OH, Halogen, -O-C₁-C₄-Alkyl, -O-C₁-C₄-Alkylen-COOH, -O-C₁-C₄-Alkylen-CO-O-C₁-C₄-Alkyl,

bedeuten, mit der Maßgabe, daß wenn R¹ und R² jeweils ortho-Methyl bedeuten, R³ nicht gleichzeitig OH sein kann, zur Herstellung eines Arzneimittels zur Behandlung von Atemwegserkrankungen, die ausgewählt sind aus der Gruppe bestehend aus Asthma bronchiale, pädiatrisches Asthme, Schweres Asthma, akuter Asthma- Anfall, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die vorstehend genannte Verwendung von Verbindungen der allgemeinen Formel **1**, worin
- n: 1 oder 2, bevorzugt 1;
- R¹: Wasserstoff, Fluor, Chlor, Methyl oder Methoxy;
- R²: Wasserstoff, Fluor, Chlor, Methyl oder Methoxy;
- R³: C₁-C₄-Alkyl, OH, Fluor, Chlor, Brom, -O-C₁-C₄-Alkyl, -O-C₁-C₄-Alkylen-COOH, -O-C₁-C₄-Alkylen-CO-O-C₁-C₄-Alkyl,
bedeuten, mit der Maßgabe, daß wenn R¹ und R² jeweils ortho-Methyl bedeuten, R³ nicht gleichzeitig OH sein kann.

Bevorzugt ist die vorstehend genannte Verwendung von Verbindungen der allgemeinen Formel 1 worin
- n: 1;
- R¹: Wasserstoff oder C₁-C₄-Alkyl;
- R²: Wasserstoff oder C₁-C₄-Alkyl;
- R³: C₁-C₄-Alkyl, OH, -O-C₁-C₄-Alkyl, -O-C₁-C₄-Alkylen-COOH oder -O-C₁-C₄- Alkylen-CO-O-C₁-C₄-Alkyl,
bedeuten, mit der Maßgabe, daß wenn R¹ und R² jeweils ortho-Methyl bedeuten, R³ nicht gleichzeitig OH sein kann.

Bevorzugt ist die vorstehend genannte Verwendung von Verbindungen der allgemeinen Formel **1**, worin
- n: 1;
- R¹: Wasserstoff, Methyl oder Ethyl;
- R²: Wasserstoff, Methyl oder Ethyl;
- R³: Methyl, Ethyl, OH, Methoxy, Ethoxy, -O-CH₂-COOH, -O-CH₂-COOMethyl oder -O-CH₂-COOEthyl,
bedeuten, mit der Maßgabe, daß wenn R¹ und R² jeweils ortho-Methyl bedeuten, R³ nicht gleichzeitig OH sein kann.

Bevorzugt ist die vorstehend genannte Verwendung von Verbindungen der allgemeinen Formel **1**, worin
- n: 1;
- R¹: Wasserstoff oder Methyl;
- R²: Wasserstoff oder Methyl;
- R³: Methyl, OH, Methoxy, -O-CH₂-COOH oder -O-CH₂-COOEthyl,
bedeuten, mit der Maßgabe, daß wenn R¹ und R² jeweils ortho-Methyl bedeuten, R³ nicht gleichzeitig OH sein kann.

Erfindungsgemäß bevorzugt ist die vorstehend genannte Verwendung von Verbindungen der allgemeinen Formel **1**, worin
- R³: Methoxy, Ethoxy, -O-CH₂-COOH, -O-CH₂-COOMethyl oder -O-CH₂-COOEthyl,
bedeuten und R¹, R² und n die vorstehend genannten Bedeutungen haben können.

Die vorliegende Erfindung betrifft ferner die vorstehend genannte Verwendung von Verbindungen der allgemeinen Formel **1**,
worin
- n: 1;
- R¹: Halogen, C₁-C₄-Alkyl oder -O-C₁-C₄-Alkyl;
- R²: Halogen, C₁-C₄-Alkyl oder -O-C₁-C₄-Alkyl;
- R³: Halogen, C₁-C₄-Alkyl oder -O-C₁-C₄-Alkyl, bedeuten.

Die vorliegende Erfindung betrifft ferner die vorstehend genannte Verwendung von Verbindungen der allgemeinen Formel **1**,
worin
- n: 1;
- R¹: Fluor, Chlor, Methyl oder Methoxy;
- R²: Fluor, Chlor, Methyl oder Methoxy ;
- R³: Fluor, Chlor, Methyl oder Methoxy bedeuten.

Ein weiterer bevorzugter Aspekt der vorliegenden Erfindung ist die vorstehend genannte Verwendung von Verbindungen der allgemeinen Formel **1** worin
- n: 1;
- R¹: Wasserstoff;
- R²: Wasserstoff, Fluor, Chlor oder Methyl;
- R³: Methyl, Ethyl, iso-Propyl, tert.-Butyl, OH, Fluor, Chlor, Brom, Methoxy, Ethoxy, -O-CH₂-COOH, -O-CH₂-CH₂-COOH, -O-CH₂-CH₂-CH₂-COOH, -O-CH₂-COOMethyl, -O-CH₂-COOEthyl, -O-CH₂-CH₂-COOMethyl, -O-CH₂-CH₂-COOEthyl, -O-CH₂-CH₂-CH₂-COOMethyl, -O-CH₂-CH₂-CH₂-COOEthyl, bedeuten.

Besonders bevorzugt ist die vorstehend genannte Verwendung von Verbindungen der allgemeinen Formel **1** worin
- n: 1;
- R¹: Wasserstoff;
- R²: Wasserstoff, Fluor, Chlor oder Methyl;
- R³: OH, Fluor, Chlor, Methyl, Methoxy, Ethoxy oder -O-CH₂-COOH, bedeuten.

Ferner ist die vorstehend genannte Verwendung von Verbindungen der allgemeinen Formel **1** erfindungsgemäß besonders bevorzugt, wenn
- n: 1;
- R¹: Wasserstoff;
- R²: Halogen, C₁-C₄-Alkyl oder -O-C₁-C₄-Alkyl, bevorzugt Fluor, Chlor, Methoxy oder Methyl;
- R³: Halogen, C₁-C₄-Alkyl oder -O-C₁-C₄-Alkyl, bevorzugt Fluor, Chlor, Methoxy oder Methyl, bedeuten.

Ein weiterer bevorzugter Aspekt der vorliegenden Erfindung ist die vorstehend genannte Verwendung von Verbindungen der allgemeinen Formel **1** in denen n = 1 , R¹ und R² Wasserstoff und der Rest R³ die vorstehend genannten Bedeutungen haben kann.

Ein weiterer bevorzugter Aspekt der vorliegenden Erfindung ist die vorstehend genannte Verwendung von Verbindungen der allgemeinen Formel **1**, worin
- n: 1;
- R¹ und R²: Wasserstoff;
- R³: Methyl, Ethyl, iso-Propyl, tert.-Butyl, OH, Fluor, Chlor, Brom, Methoxy, Ethoxy, -O-CH₂-COOH, -O-CH₂-CH₂-COOH, -O-CH₂-CH₂-CH₂-COOH, -O-CH₂-COOMethyl, -O-CH₂-COOEthyl, -O-CH₂-CH₂-COOMethyl, -O-CH₂-CH₂-COOEthyl, -O-CH₂-CH₂-CH₂-COOMethyl , -O-CH₂-CH₂-CH₂-COOEthyl, bedeuten.

Besonders bevorzugt ist die vorstehend genannte Verwendung von Verbindungen der allgemeinen Formel **1**, worin
- n: 1;
- R¹ und R²: Wasserstoff;
- R³: OH, Fluor, Chlor, Methoxy, Ethoxy,-O-CH₂-COOH, bevorzugt OH, Fluor, Chlor, Ethoxy oder Methoxy, bedeuten.

Besonders bevorzugt ist die vorstehend genannte Verwendung von Verbindungen der allgemeinen Formel **1**, worin
- n: 1;
- R¹ und R²: Wasserstoff;
- R³: Fluor, Chlor, Methoxy oder Ethoxy bedeuten.

Die vorliegende Erfindung betrifft ferner die vorstehend genannte Verwendung von Verbindungen der allgemeinen Formel **1**, worin
- n: 1;
- R¹: Wasserstoff, Halogen, C₁-C₄-Alkyl oder -O-C₁-C₄-Alkyl;
- R²: Wasserstoff, Halogen, C₁-C₄-Alkyl oder -O-C₁-C₄-Alkyl;
- R³: Wasserstoff, bedeuten.

Bevorzugt ist dabei die vorstehend genannte Verwendung von Verbindungen der allgemeinen Formel **1,** worin
- n: 1;
- R¹: Wasserstoff, Fluor, Chlor, Methyl oder Methoxy;
- R²: Wasserstoff, Fluor, Chlor, Methyl oder Methoxy;
- R³: Wasserstoff, bedeuten.

Die vorliegende Erfindung betrifft ferner die vorstehend genannte Verwendung von Verbindungen der allgemeinen Formel 1, worin
- n: 1;
- R¹: Fluor, Chlor, Methyl oder Methoxy;
- R²: Fluor, Chlor, Methyl oder Methoxy;
- R³: Wasserstoff, bedeuten.

In den Verbindungen der Formel **1** können die Reste R¹ und R², sofern sie nicht Wasserstoff bedeuten, jeweils ortho oder meta bezüglich der Verknüpfung zur benzylischen "-CH₂"-Gruppe angeordnet sein. Wenn keiner der Reste R¹ und R² Wasserstoff bedeutet, sind für die erfindungsgemäße Verwendung diejenigen Verbindungen der Formel **1** bevorzugt, in denen beide Reste R¹ und R² entweder ortho oder beide Reste R¹ und R² meta konfiguriert sind, wobei Verbindungen, in denen beide Reste R¹ und R² ortho-konfiguriert sind, besondere Bedeutung zukommt.

In den Verbindungen der Formel **1** in denen einer der Reste R¹ und R² nicht Wasserstoff bedeutet, kann dieser ortho oder meta bezüglich der Verknüpfung zur benzylischen "-CH₂"-Gruppe angeordnet sein. In diesem Fall sind für die erfindungsgemäße Verwendung insbesondere diejenigen Verbindungen der Formel **1** bevorzugt, in denen der Rest R¹ oder R² , der nicht Wasserstoff bedeutet, ortho-konfiguriert ist.

Besonders bevorzugt ist ferner die vorstehend genannte Verwendung einer oder mehrerer der nachstehenden Verbindungen der allgemeinen Formel **1**:
- 6-Hydroxy-8-{1-hydroxy-2-[2-(4-methoxy-phenyl)-1,1-dimethyl-ethylamino]-ethyl }-4H-benzo[1,4]oxazin-3-on;
- 6-Hydroxy-8-{1-hydroxy-2-[2-(4-phenoxy-essigsäureethylester)-1,1-dimethyl-ethylamino]-ethyl}-4H-benzo[1,4]oxazin-3-on;
- 6-Hydroxy-8-{1-hydroxy-2-[2-(4-phenoxy-essigsäure)-1,1-dimethyl-ethylamino]-ethyl}-4H-benzo[1,4]oxazin-3-on;
- 8-{2-[1,1-Dimethyl-2-(2,4,6-trimethylphenyl)-ethylamino]-1-hydroxy-ethyl}-6-hydroxy-4H-benzo[1,4]oxazin-3-on;
- 6-Hydroxy-8-{1-hydroxy-2-[2-(4-hydroxy-phenyl)-1,1-dimethyl-ethylamino]-ethyl}-4H-benzo[1,4]oxazin-3 -on;
- 6-Hydroxy-8-{1-hydroxy-2-[2-(4-isopropyl-phenyl)-1,1dimethyl-ethylamino]-ethyl}-4H-benzo[1,4]oxazin-3-on;
- 8-{2-[2-(4-Ethyl-phenyl)-1,1-dimethyl-ethylamino]-1-hydroxy-ethyl}-6-hydroxy-4H-benzo[1,4]oxazin-3-on;
- 8-{2-[2-(4-Fluoro-3-methyl-phenyl)-1,1-dimethyl-ethylamino]-1-hydroxy-ethyl}-6-hydroxy-4H-benzo[1,4]oxazin-3-on;
- 8-{2-[2-(4-Fluor-2-methyl-phenyl)-1,1-dimethyl-ethylamino]-1-hydroxy-ethyl}-6-hydroxy-4H-benzo[1,4]oxazin-3-on;
- 8-{2-[2-(2,4-Difluor-phenyl)-1,1-dimethyl-ethylamino]-1-hydroxy-ethyl}-6-hydroxy-4H-benzo[1,4]oxazin-3-on;
- 8-{2-[2-(3,5-Difluor-phenyl)-1,1-dimethyl-ethylamino]-1-hydroxy-ethyl}-6-hydroxy-4H-benzo[1,4]oxazin-3-on;
- 8-{2-[2-(4-Ethoxy-phenyl)-1,1-dimethyl-ethylamino]-1-hydroxy-ethyl}-6-hydroxy-4H-benzo[1,4]oxazin-3-on;
- 8-{2-[2-(3,5-Dimethyl-phenyl)-1,1-dimethyl-ethylamino]-1-hydroxy-ethyl}-6-hydroxy-4H-benzo[1,4]oxazin-3-on;
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure;
- 8-{2-[2-(3,4-Difluor-phenyl)-1,1-dimethyl-ethylamino]-1-hydroxy-ethyl}-6-hydroxy-4H-benzo[1,4]oxazin-3-on;
- 8-{2-[2-(2-Chlor-4-fluor-phenyl)-1,1-dimethyl-ethylamino]-1-hydroxy-ethyl}-6-hydroxy-4H-benzo[1,4]oxazin-3-on;
- 8-{2-[2-(4-Chlor-phenyl)-1,1-dimethyl-ethylamino]-1-hydroxy-ethyl}-6-hydroxy-4H-benzo[1,4]oxazin-3-on;
- 8-{2-[2-(4-Brom-phenyl)-1,1-dimethyl-ethylamino]-1-hydroxy-ethyl}-6-hydroxy-4H-benzo[1,4]oxazin-3-on;
- 8-{2-[2-(4-Fluor-phenyl)-1,1-dimethyl-ethylamino]-1-hydroxy-ethyl}-6-hydroxy-4H-benzo[1,4]oxazin-3-on;
- 8-{2-[2-(4-Fluor-3-methoxy-phenyl)-1,1-dimethyl-ethylamino]-1-hydroxy-ethyl}-6-hydroxy-4H-benzo[1,4]oxazin-3-on;
- 8-{2-[2-(4-Fluor-2,6-dimethyl-phenyl)-1,1-dimethyl-ethylamino]-1-hydroxy-ethyl}-6-hydroxy-4H-benzo[1,4]oxazin-3-on;
- 8-{2-[2-(4-Chlor-2-methyl-phenyl)-1,1-dimethyl-ethylamino]-1-hydroxy-ethyl}-6-hydroxy-4H-benzo[1,4]oxazin-3-on;
- 8-{2-[2-(4-Chlor-3-fluor-phenyl)-1,1-dimethyl-ethylamino]-1-hydroxy-ethyl}-6-hydroxy-4H-benzo[1,4]oxazin-3-on;
- 8-{2-[2-(4-Chlor-2-fluor-phenyl)-1,1-dimethyl-ethylamino]-1-hydroxy-ethyl}-6-hydroxy-4H-benzo[1,4]oxazin-3-on;
- 8-{2-[2-(3-Chlor-4-fluor-phenyl)-1,1-dimethyl-ethylamino]-1-hydroxy-ethyl}-6-hydroxy-4H-benzo[1,4]oxazin-3-on;
- 8-{2-[2-(2,6-Difluor-4-methoxy-phenyl)-1,1-dimethyl-ethylamino]-1-hydroxyethyl}-6-hydroxy-4H-benzo[1,4]oxazin-3-on;
- 8-{2-[2-(2,5-Difluor-4-methoxy-phenyl)-1,1-dimethyl-ethylamino]-1-hydroxyethyl}-6-hydroxy-4H-benzo[1,4]oxazin-3-on;
- 8-{2-[2-(4-Fluor-3,5-dimethyl-phenyl)-1,1-dimethyl-ethylamino]-1-hydroxy-ethyl}-6-hydroxy-4H-benzo[1,4]oxazin-3-on;
- 8-{2-[2-(3,5-Dichlor-phenyl)-1,1-dimethyl-ethylamino]-1-hydroxy-ethyl}-6-hydroxy-4H-benzo[1,4]oxazin-3-on;
- 8-{2-[2-(4-Chlor-3-methyl-phenyl)-1,1-dimethyl-ethylamino]-1-hydroxy-ethyl)-6-hydroxy-4H-benzo[1,4]oxazin-3-on;
- 8-{2-[2-(3,4,5-Trifluor-phenyl)-1,1-dimethyl-ethylamino]-1-hydroxy-ethyl}-6-hydroxy-4H-benzo[1,4]oxazin-3-on;
- 8-{2-[2-(3-Methyl-phenyl)-1,1-dimethyl-ethylamino]-1-hydroxy-ethyl}-6-hydroxy-4H-benzo[1,4]oxazin-3-on und
- 8-{2-[2-(3,4-Dichlor-phenyl)-1,1-dimethyl-ethylamino]-1-hydroxy-ethyl}-6-hydroxy-4H-benzo[1,4]oxazin-3-on.

Ein weiterer Aspekt der vorliegenden Erfindung betrifft die vorstehend genannte Verwendung der Verbindungen der Formel **1** in Form der einzelnen optischen Isomeren, Mischungen der einzelnen Enantiomeren oder Racemate. Besonders bevorzugt ist hierbei die vorstehend genannte Verwendung der Verbindungen der Formel **1** in Form der enantiomerenreinen Verbindungen, wobei die Verwendung der R-Enantiomere der Verbindungen der Formel **1** erfindungsgemäß von herausragender Bedeutung ist.

Ein weiterer Aspekt der vorliegenden Erfindung betrifft die vorstehend genannte Verwendung der Verbindungen der Formel **1** in Form der Säureadditionssalze mit pharmakologisch unbedenklichen Säuren sowie gegebenenfalls in Form der Solvate und/oder Hydrate.

Bevorzugt ist die Verwendung von Verbindungen der allgemeinen Formel **1** zur Herstellung eines Arzneimittels zur Behandlung von Obstruktive Lungenerkrankungen die ausgewählt sind aus der Gruppe bestehend aus Asthma Bronchiale, pädiatrisches Asthma, schweres Asthma, akuter Asthma-Anfall, wobei die Verwendung zur Herstellung eines Arzneimittels zur Behandlung von Asthma Bronchiale erfindungsgemäß besonders bevorzugt ist.

Bevorzugt ist ferner die Verwendung von Verbindungen der allgemeinen Formel **1** zur Herstellung eines Arzneimittels zur Behandlung von Lungenemphysemen, die ihren Ursprung haben in α1-Proteinase-Inhibitor-Mangel.

Bevorzugt ist ferner die Verwendung von Verbindungen der allgemeinen Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, brochoalveoläres Karzinom und Lymphome.

Bevorzugt ist ferner die Verwendung von Verbindungen der allgemeinen Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung von Verbindungen der allgemeinen Formel **1** zur Herstellung eines Arzneimittels zur Behandlung von Zystischer Fibrose bzw. Mukoviszidose.

Bevorzugt ist ferner die Verwendung von Verbindungen der allgemeinen Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung von Verbindungen der allgemeinen Formel **1** zur Herstellung eines Arzneimittels zur Behandlung von Bronchiektasen.

Bevorzugt ist ferner die Verwendung von Verbindungen der allgemeinen Formel **1** zur Herstellung eines Arzneimittels zur Behandlung von ARDS (adult respiratory distress syndrom).

Bevorzugt ist ferner die Verwendung von Verbindungen der allgemeinen Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der Verbindungen der Formel **1** zur Herstellung eines Arzneimittels zur Behandlung von Asthma. Von besonderer Bedeutung ist ferner die vorstehend genannte Verwendung von Verbindungen der Formel 1 zur Herstellung eines Arzneimittels zur einmal täglichen Behandlung von entzündlichen und obstruktiven Atemwegserkrankungen ausgewählt aus der Gruppe bestehend aus Asthma Bronchiale, pädiatrisches Asthma, schweres Asthma, akutes Asthma Anfall, besonders bevorzugt zur einmal täglichen Behandlung von Asthma.

Ferner betrifft die vorliegende Erfindung ein Verfahren zur Behandlung der vorstehend genannten Erkrankungen, dadurch gekennzeichnet, daß eine oder mehrere der vorstehend genannten Verbindungen der allgemeinen Formel **1** in therapeutisch wirksamen Mengen appliziert werden. Die vorliegende Erfindung betrifft ferner Verfahren zur Behandlung von Asthma, dadurch gekennzeichnet, daß eine oder mehrere der vorstehend genannten Verbindungen der allgemeinen Formel **1** in therapeutisch wirksamen Mengen einmal täglich appliziert werden.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Zur erfindungsgemäßen Verwendung können die Verbindungen der allgemeinen Formel **1** gegebenenfalls in Form der einzelnen optischen Isomeren, Mischungen der einzelnen Enantiomeren oder Racemate zur Anwendung gelangen. Werden die Verbindungen in enantiomerenreiner Form eingesetzt, werden bevorzugt die R-Enantiomere verwendet.

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, n-Propylen oder n-Butylen.

Als Alkyloxygruppen (oder auch -O-Alkylgruppen) werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Die Herstellung der erfindungsgemäßen Verbindungen kann in Analogie zu im Stand der Technik bereits bekannten Vorgehensweisen erfolgen. Geeignete Herstellverfahren sind beispielsweise aus der US 4460581 bekannt, auf die an dieser Stelle vollinhaltlich Bezug genommen wird.

Die nachstehend beschriebenen Synthesebeispiele dienen der weitergehenden Illustration der Erfindung. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Beispiel 1: 6-Hydroxy-8-{1-hydroxy-2-[2-(4-methoxy-phenyl)-1,1-dimethyl-ethylamino]-ethyl}-4H-benzo[1,4]oxazin-3-on

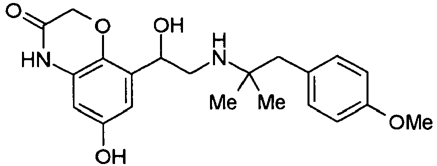

### a) 8-{2-[1,1-Dimethyl-2-(4-methoxy-phenyl)-ethylamino]-1-hydroxy-ethyl)-6-benzyloxy-4H-benzo[1,4]oxazin-3-on

Zu einer Lösung von 3.6 g 1,1-Dimethyl-2-(4-methoxyphenyl)-ethylamin in 100 mL Ethanol werden bei 70°C 7.5 g (6-Benzyloxy-4H-benzo[1,4]oxazin-3-on)-glyoxalhydrat gegeben und man läßt 15 Minuten rühren. Anschließend werden innerhalb von 30 Minuten bei 10 bis 20°C 1 g Natriumborhydrid zugesetzt. Es wird eine Stunde gerührt, mit 10 mL Aceton versetzt und über weitere 30 Minuten gerührt. Die Reaktionsmischung wird mit 150 mL Ethylacetat verdünnt, mit Wasser gewaschen, mit Natriumsulfat getrocknet und eingeengt. Der Rückstand wird in 50 mL Methanol und 100 mL Ethylacetat gelöst und mit konz. Salzsäure sauer gestellt. Nach Zugabe von 100 mL Diethylether fällt das Produkt aus. Die Kristalle werden abfiltriert, gewaschen und in 50 mL Ethanol umkristallisiert. Ausbeute: 7 g (68%; Hydrochlorid); Schmp. = 232-234°C.

### b) 8-{2-[1,1-Dimethyl-2-(4-methoxy-phenyl)-ethylamino]-1-hydroxy-ethyl}-6-hydroxy-4H-benzo[1,4]oxazin-3-on

6.8 g der vorstehend erhaltenen Benzylverbindung werden in 125 mL Methanol unter Zusatz von 1 g Palladium auf Kohle (5%ig) bei Raumtemperatur und Normaldruck hydriert. Der Katalysator wird abfiltriert und das Filtrat vom Lösungsmittel befreit. Nach Umkristallisation des Rückstandes in 50 mL Aceton und etwas Wasser wird ein Feststoff erhalten, der abfiltriert und gewaschen wird.
Ausbeute: 5.0 g (89 %; Hydrochlorid); Schmp. = 155-160°C.

Die (R)- und (S)-Enantiomere von Beispiel 1 können aus dem Racemat beispielsweise mittels chiraler HPLC erhalten werden (z.B. Säule: Chirobiotic T, 250 x 22.1 mm von der Firma Astec). Als mobile Phase kann Methanol mit 0.05 % Triethylamin und 0.05% Essigsäure verwendet werden. Kieselgel mit einer Korngröße von 5 µm, an das kovalent das Glykoprotein Teicoplanin gebunden ist, kann als Säulenmaterial zum Einsatz gelangen. Retentionszeit (R-Enantiomer) = 40.1 min, Retentionszeit (S-Enatiomer) = 45.9 min. Die beiden Enantiomere werden nach dieser Methode in Form der freien Basen erhalten. Erfindungsgemäß von herausragender Bedeutung ist das R-Enantiomer des Beispiels 1.

### Beispiel 2: 6-Hydroxy-8-{1-hydroxy-2-[2-(4-phenoxy-essigsäureethylester)-1,1-dimethyl-ethylamino]-ethyl}-4H-benzo[1,4]oxazin-3-on

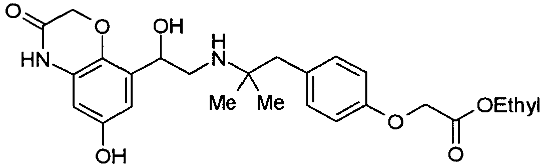

### a) 8-{2-[1,1-Dimethyl-2-(4-phenoxy-essigsäureethylester)-ethylamino]-1-hydroxy-ethyl}-6-benzyloxy-4H-benzo[1,4]oxazin-3-on

In Analogie zur unter Beispiel 1a) beschriebenen Vorgehensweise wird aus 15 g (6-Benzyloxy-4H-benzo[1,4]oxazin-3-on)-glyoxalhydrat und 11.8 g 1,1-Dimethyl-2-(4-phenoxy-essigsäureethylester)-ethylamin Hydrochlorid die Titelverbindung erhalten. Ausbeute: 16.5 g (69%, Hydrochlorid); Schmp. = 212-214°C.

### b) 8-{2-[1,1-Dimethyl-2-(4-phenoxy-essigsäureethylester)-ethylamino]-1-hydroxy-ethyl}-6-hydroxy-4H-benzo[1,4]oxazin-3-on

8 g des vorstehend erhaltenen Benzylalkohols werden in 100 mL Ethanol, 100 mL Methanol und 10 mL Wasser gelöst und in Gegenwart von 1 g Palladium auf Kohle (5%ig) hydriert. Nach Aufnahme der theoretisch berechneten Menge Wasserstoff wird der Katalysator abfiltriert und das Filtrat eingeengt. Das beim Abdestillieren der Lösungsmittel auskristallisierende Produkt wird abgesaugt und gewaschen.
Ausbeute: 5.5 g (81%; Hydrochlorid); Schmp. = 137-140°C.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 3: 6-Hydroxy-8-{1-hydroxy-2-[2-(4-phenoxy-essigsäure)-1,1-dimethyl-ethylamino]-ethyl}-4H-benzo[1,4]oxazin-3-on

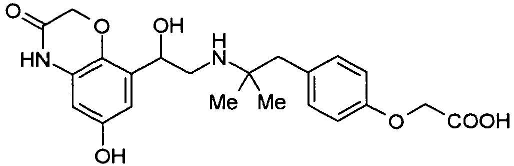

11 g 8-{2-[1,1-Dimethyl-2-(4-phenoxy-essigsäureethylester)-ethylamino]-1-hydroxyethyl}-6-benzyloxy-4H-benzo[1,4]oxazin-3-on hydrochlorid (Beispiel 4a) werden in 125 mL Methanol gelöst und in Gegenwart von 1 g Palladium auf Kohle (5%ig) hydriert. Nach Aufnahme der theoretisch berechneten Menge an Wasserstoff wird der Katalysator abfiltriert. Zum Filtrat werden 2.6 g Natriumhydroxid gelöst in 20 mL Wasser gegeben. Man läßt 30 Minuten refluxieren, destilliert das Methanol ab und versetzt mit 10 mL Wasser, 20 mL n-Butanol und 3.9 mL Essigsäure. Der ausfallende Feststoff wird abgesaugt und mit Diethylether gewaschen.
Ausbeute: 7 g (87%). Durch Umkristallisation aus 0.5 molarer Salzsäure wird das Hydrochlorid erhalten. Schmp. = 152°C.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 4: 8-{2-[1,1-Dimethyl-2-(2,4,6-trimethylphenyl)-ethylamino]-1-hydroxy-ethyl}-6-hydroxy-4H-benzo[1,4]oxazin-3-on

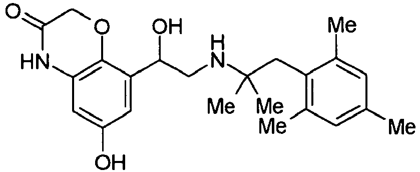

### a) 1-(6-Benzyloxy-4H-benzo[1,4]oxazin-3-on)-2-[1,1-dimethyl-2-(2,4,6-trimethylphenyl)-ethylimino]-ethanon

7.2 g (6-Benzyloxy-4H-benzo[1,4]oxazin-3-on)-glyoxalhydrat und 3.6 g 1,1-Dimethyl-2-(2,4,6-trimethylphenyl)-ethylamin werden eine Stunde in 100 mL Ethanol auf 70°C erwärmt. Nach dem Abkühlen wird der ausgefallenen Feststoff abfiltriert und mit Ethanol und Diethylether gewaschen. Ausbeute: 8.6 g (94%); Schmp. = 175°C.

### b) 8-{2-[1,1-Dimethyl-2-(2,4,6-trimethylphenyl)-ethylamino]-1-hydroxy-ethyl}-6-benzyloxy-4H-benzo[1,4]oxazin-3-on

8.6 g der nach der Vorschrift 6a) erhaltenen Schiffschen Base werden in 100 mL Ethanol und 20 mL THF gelöst, innerhalb von 30 min bei 10-20°C mit 0.7 g Natriumborhydrid versetzt und eine Stunde gerührt. Nach Zugabe von 10 mL Aceton wird 30 Minuten nachgerührt und dann mit Ethylacetat und Wasser verdünnt. Das beim Ansäuern mit konz. Salzsäure auskristallisierende Produkt wird abfiltriert und gewaschen.
Ausbeute: 7.4 g (80%, Hydrochlorid); Schmp. = 235°C (Zersetzung).

### c) 8-{2-[1,1-Dimethyl-2-(2,4,6-trimethylphenyl)-ethylamino]-1-hydroxy-ethyl}-6-hydroxy-4H-benzo[1,4]oxazin-3-on

7.4 g der unter Stufe b) erhaltenen Benzylverbindung werden in 125 mL Methanol unter Zusatz von 1 g Palladium auf Kohle (5%ig) bei Raumtemperatur und Normaldruck hydriert. Anschließend wird der Katalysator abfiltriert und das Filtrat eingeengt. Das bei Zugabe von Aceton auskristallisierende Produkt wird abgesaugt und mit Aceton und Diethylether gewaschen. Ausbeute: 5 g (78%, Hydrochlorid); Schmp. = 160°C (Zersetzung).

Die (R)- und (S)-Enantiomere dieses Ausführungsbeispiels können durch Auftrennung des Racemats in Analogie zu gängigen, im Stand der Technik bekannten Methoden erhalten werden.

### Beispiel 5: 6-Hydroxy-8-{1-hydroxy-2-[2-(4-hydroxy-phenyl)-1,1-dimethyl-ethylamino]-ethyl}-4H-benzo[1,4]oxazin-3-on

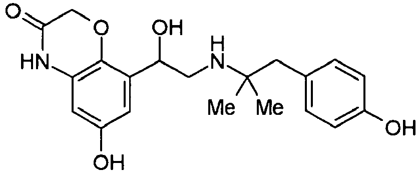

### a) 8-{2-[1,1-Dimethyl-2-(4-hydroxy-phenyl)-ethylamino]-1-hydroxy-ethyl}-6-benzyloxy-4H-benzo[1,4]oxazin-3-on

Die Titelverbindung wird aus 10 g (6-Benzyloxy-4H-benzo[1,4]oxazin-3-on)-glyoxalhydrat und 4.6 g 1,1-Dimethyl-2-(4-hydroxy-phenyl)-ethylamin analog zur Vorschrift für Beispiel 1a) hergestellt.
Ausbeute: 9.0 g (64%, Hydrochlorid); Schmp. = 255-258°C.

### b) 8-{2-[1,1-Dimethyl-2-hydroxy-phenyl)-ethylamino]-1-hydroxy-ethyl}-6-hydroxy-4H-benzo[1,4]oxazin-3-on

5.7 g des vorstehend erhaltenen Kupplungsproduktes werden in Gegenwart von 0.6 g Palladium auf Kohle (5%ig) in 100 mL Methanol hydriert. Nach Aufnahme der theoretisch berechneten Menge an Wasserstoff wird der Katalysator abfiltriert und das Filtrat vom Lösungsmittel befreit. Der Rückstand wird unter Erwärmung in Ethanol gelöst und dann mit Diethylether versetzt. Das ausfallende Produkt wird abgesaugt und einmal in Wasser umkristallisiert. Ausbeute: 3.6 g (72%, Hydrochlorid); Schmp. = 159-162°C.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 6: 6-Hydroxy-8-{1-hydroxy-2-[2-(4-isopropyl-phenyl)-1,1dimethyl-ethylamino]-ethyl}-4H-benzo[1,4]oxazin-3-on

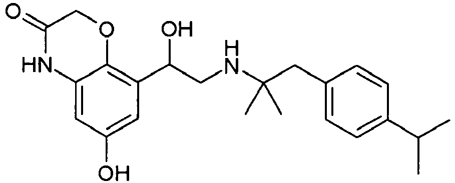

### a) 1-(4-Isopropyl-phenyl)-2-methyl-propan-2-ol

Die Umsetzung einer Grignardverbindung, hergestellt aus 20 g (119 mmol) 4-Isopropylbenzylchlorid, mit 11.4 ml (155 mmol) Aceton liefert die Zielverbindung als farbloses Öl. Ausbeute: 13.0 g (57%); Massenspekrometrie: [M+H]⁺ = 193.

### b) N-[2-(4-Isopropyl-phenyl)-1,1-dimethyl-ethyl]-acetamid

Es wird eine Ritter-Reaktion mit 10.2 g (53 mmol) 1-(4-Isopropyl-phenyl)-2-methyl-propan-2-ol in der für Beispiel 7b) beschriebenen Weise durchgeführt. Das Reaktionsgemisch wird auf Eiswasser gegossen und mit Natronlauge alkalisch gestellt, wobei ein Feststoff ausfällt. Dieser wird abgesaugt und getrocknet. Ausbeute: 9.90 g (80%); Massenspektrometrie: [M+H]⁺ = 234.

### c) 2-(4-Isopropyl-phenyl)-1,1-dimethyl-ethylamin

Umsetzung von 9.80 g (42 mmol) N-[2-(4-Isopropyl-phenyl)-1,1-dimethyl-ethyl]-acetamid in Analogie zur Vorschrift für Beispiel 7c). Ausbeute: 7.00 g (71%, Hydrochlorid); Smp. = 202-206°C.

### d) 6-Benzyloxy-8-{1-hydroxy-2-[2-4-isopropyl-phenyl)-1,1-dimethyl-ethylamino]-ethyl}-4H-benzo[1,4]oxazin-3-on

2.18 g (6.1 mmol) Benzyloxy-8-(2-ethoxy-2-hydroxy-acetyl)-4H-benzo[1,4]oxazin-3-on und 1.1 g (5.8 mmol) 2-(4-Isopropyl-phenyl)-1,1-dimethyl-ethylamin werden eine Stunde bei 50-80°C in 40 mL Ethanol gerührt. Nach Abkühlung auf Raumtemperatur werden 0.24 g (6.3 mmol) Natriumborhydrid zugesetzt. Man läßt eine Stunde rühren, verdünnt mit 5 mL Aceton und läßt weitere 30 Minuten rühren. Die Reaktionsmischung wird mit Salzsäure angesäuert, mit 100 mL Wasser und 80 mL Ethylacetat versetzt und mit Ammoniak alkalisch gestellt. Die organische Phase wird abgetrennt, mit Natriumsulfat getrocknet und vom Lösungsmittel befreit. Der Rückstand wird in 20 mL Ethylacetat und 10 mL Wasser gelöst, mit konz. Salzsäure sauer gestellt und mit Diethylether verdünnt. Nach Zugabe einer Kristallisationshilfe wird der ausfallende Feststoff abgesaugt und gewaschen. Weißer Feststoff. Ausbeute: 1.7 g (52 %, Hydrochlorid); Smp. = 220-222°C.

### e) 6-Hydroxy-8-{1-hydroxy-2-[2-(4-isopropyl-phenyl)-1,1dimethyl-ethylamino]-ethyl}-4H-benzo[1,4]oxazin-3-on

1.6 g (3.0 mmol) 6-Benzyloxy-8-{1-hydroxy-2-[2-(4-isopropyl-phenyl)-1,1-dimethyl-ethylamino]-ethyl}-4H-benzo[1,4]oxazin-3-on werden in Methanol gelöst und mit Palladium auf Kohle als Katalysator bei Normaldruck und Raumtemperatur hydriert. Der Katalysator wird abgesaugt, das Lösungsmittel abdestilliert und der Rückstand in Isopropanol auskristallisiert. Weißer Feststoff. Ausbeute: 1.1 g (85%, Hydrochlorid); Smp. = 248-250°C; Massenspektrometrie: [M+H]⁺ = 399.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 7: 8-{2-[2-(4-Ethyl-phenyl)-1,1-dimethyl-ethylamino]-1-hydroxy-ethyl}-6-hydroxy-4H-benzo[1,4]oxazin-3-on

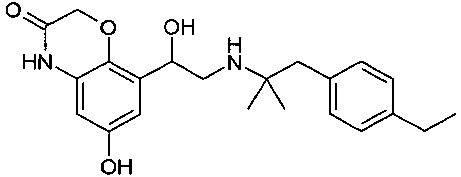

### a) 1-(4-Ethyl-phenyl)-2-methyl-propan-2-ol

Zu 39 mL einer 3 molaren Lösung von Methylmagnesiumbromid in Diethylether werden unter Kühlung mit dem Eisbad 14.8 g (90 mmol) 1-(4-Ethyl-phenyl)-propan-2-on, gelöst in Diethylether, so zugetropft, dass die Temperatur nicht über 30°C steigt. Nach beendeter Zugabe lässt man die Reaktionsmischung 1.5 Stunden refluxieren und hydrolisiert dann mit 10%iger Ammnoniumchlorid-Lösung. Nach Abtrennung der organischen Phase wird die wässrige Phase mit Diethylether extrahiert. Die vereinigten Etherphasen werden mit Wasser gewaschen, mit Natriumsulfat getrocknet und eingeengt. Das so erhaltene Öl wird direkt weiter umgesetzt. Ausbeute: 15.5 g (90%).

### b) N-[2-(4-Ethyl-phenyl)-1,1-dimethyl-ethyl]-acetamid

Zu 15.5 g (87 mmol) 1-(4-Ethyl-phenyl)-2-methyl-propan-2-ol in 4.8 mL (91 mmol) Acetonitril und 15 mL Eisessig werden innerhalb von 15 Minuten 6.2 mL konz. Schwefelsäure zugetropft, wobei die Temperatur auf 65°C ansteigt. Anschließend wird eine Stunde gerührt, mit Eiswasser verdünnt und mit konz. Natronlauge alkalisch gestellt. Nach weiteren Rühren über 30 Minuten wird der ausgefallene Feststoff abgesaugt und mit Wasser gewaschen. Das Rohprodukt wird in Ethylacetat gelöst, mit Natriumsulfat getrocknet und eingeengt. Das zurückbleibende Öl versetzt man mit Petrolether, wobei ein Feststoff ausfällt, der abfiltriert und getrocknet wird. Ausbeute: 16.3 g (85%); Schmp. = 90-92°C.

### c) 2-(4-Ethyl-phenyl)-1,1-dimethyl-ethylamin

16.3 g (74 mmol) N-[2-(4-Ethyl-phenyl)-1,1-dimethyl-ethyl]-acetamid und 8.0 g Kaliumhydroxid werden über 15 Stunden in 60 mL Ethylenglykol unter Rückfluß erhitzt. Die Reaktionsmischung wird mit Eiswasser versetzt und dreimal mit Diethylether extrahiert. Die vereinigten organischen Phasen werden mit Wasser gewaschen, mit Natriumsulfat getrocknet und vom Lösungsmittel befreit. Zur Herstellung des Hydrochlorids wird das Rohprodukt in Acetonitril gelöst und nacheinander mit etherischer Salzsäure und Diethylether versetzt. Der ausfallende Feststoff wird abgesaugt und getrocknet. Ausbeute: 11.0 g (69%, Hydrochlorid); Schmp. = 165-167°C.

### d) 6-Benzyloxy-8-{2-[2-(4-ethyl-phenyl)-1,1-dimethyl-ethylamino]-1-hydroxy-ethyl}-4H-benzo[1,4]oxazin-3-on

Die Zielverbindung wird in Analogie zur Vorschrift für Beispiel 6d) aus 2.14 g (6.0 mmol) 6-Benzyloxy-8-(2-ethoxy-2-hydroxy-acetyl)-4H-benzo[1,4]oxazin-3-on und 1.0 g (5.6 mmol) 2-(4-Ethyl-phenyl)-1,1-dimethyl-ethylamin hergestellt. Weißer Feststoff. Ausbeute: 1.7 g (54%, Hydrochlorid); Smp. 210-214°C.

### e) 8-{2-[2-(4-Ethyl-phenyl)-1,1-dimethyl-ethylamino]-1-hydroxy-ethyl}-6-hydroxy-4H-benzo[1,4]oxazin-3-on

Die Hydrogenolyse von 1.45 g (2.75 mmol) 6-Benzyloxy-8-{2-[2-(4-ethyl-phenyl)-1,1-dimethyl-ethylamino]-1-hydroxy-ethyl}-4H-benzo[1,4]oxazin-3-on nach der Vorschrift für Beispiel 6e) liefert die Zielverbindung in Form eines weißen Feststoffs. Ausbeute: 1.07 g (92%; Hydrochlorid); Smp. = 266-269°C; Massenspektrometrie: [M+H]⁺ = 385.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 8: 8-{2-[2-(4-Fluoro-3-methyl-phenyl)-1,1-dimethyl-ethylamino]-1-hydroxyethyl}-6-hydroxy-4H-benzo[1,4]oxazin-3-on

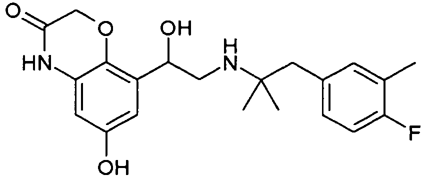

### a) 1-Fluor-2-methyl-4-(2-methyl-propenyl)-benzol

100 mL einer 0.5 molaren Lösung von 4-Fluor-3-methyl-phenylmagnesiumbromid in THF werden innerhalb von 30 Minuten mit 4.7 mL (50 mmol) Isopropylaldehyd versetzt, wobei die Temperatur auf 45°C ansteigt. Es wird 30 Minuten gerührt, 1 Stunde refluxiert und dann mit 10%iger Ammoniumchlorid-Lösung hydrolisiert. Nach Abtrennung der organischen Phase extrahiert man mit Diethylether. Die organischen Phasen werden vereinigt, getrocknet und eingeengt. Der so erhaltene Alkohol wird in 100 mL Toluol gelöst, mit 1 gp-Toluensulfonsäure Monohydrat versetzt und drei Stunden am Wasserabscheider unter Rückfluß erwärmt. Die Reaktionsmischung wird auf Wasser gegossen und mit konz. Natronlauge alkalisch gestellt. Nach Abtrennung der organischen Phase wird diese mit Wasser gewaschen, mit Natriumsulfat getrocknet und vom Lösungsmittel befreit. Eine fraktionierte Destillation des Rückstandes liefert das Produkt in Form einer farblosen Flüssigkeit (Sdp. 80-85°C/10 mbar). Ausbeute: 4.1 g (50%).

### b) N-[2-(4-Fluor-3-methyl-phenyl)-1,1-dimethyl-ethyl]-formamid

Zu 1.5 g (31 mmol) Natriumcyanid in 5 mL Eisessig werden bei 5-15°C 4.9 mL konz. Schwefelsäure getropft. Anschließend wird die Mischung mit 3.9 g (24 mmol) 1-Fluor-2-methyl-4-(2-methyl-propenyl)-benzen, gelöst in 10 mL Eisessig, versetzt und 1 Stunde bei 50-60°C gerührt. Die Reaktionsmischung wird mit Eiswasser verdünnt, mit konz. Natronlauge alkalisch gestellt und mit Dichlormethan extrahiert. Die organische Phase wird mit Natriumsulfat getrocknet und im Vakuum vom Lösungsmitteln befreit. Das so erhaltene leicht gelbe Öl wird direkt weiter umgesetzt. Ausbeute: 4.3 g (87%).

### c) 2-(4-Fluor-3-methyl-phenyl)-1,1-dimethyl-ethylamin

4.3 g (20.6 mmol) N-[2-(4-Fluor-3-methyl-phenyl)-1,1-dimethyl-ethyl]-formamid, 20 mL konz. Salzsäure und 20 mL Wasser werden über 2 Stunden unter Rückfluß erwärmt. Man verdünnt die Reaktionsmischung mit Wasser, stellt mit konz. Natronlauge alkalisch und extrahiert mit Dichlormethan. Die organischen Phasen werden mit Natriumsulfat getrocknet und eingeengt. Der Rückstand wird in Ethylacetat gelöst, mit etherischer Salzsäure versetzt und gekühlt. Die ausfallenden Kristalle werden abgesaugt und mit Diethylether gewaschen und getrocknet. Weißer Feststoff. Ausbeute: 3.9 g (87%, Hydrochlorid); Schmp. =196-198°C.

### d) 6-Benzyloxy-8-{2-[2-(4-fluor-3-methyl-phenyl)-1,1-dimethyl-ethylamino]-1-hydroxyethyl}-4H-benzo[1,4]oxazin-3-on

1.10 g (3.1 mmol) Benzyloxy-8-(2-ethoxy-2-hydroxy-acetyl)-4H-benzo[1,4]oxazin-3-on und 0.50 g (2.8 mmol) 2-(4-Fluor-3-methyl-phenyl)-1,1-dimethyl-ethylamin werden analog zu der Vorschrift für Beispiel 6d) umgesetzt und aufgearbeitet. Weißer Feststoff. Ausbeute: 0.75 g (47%, Hydrochlorid); Smp. = 228-230°C.

### e) 8-{2-[2-(4-Fluoro-3-methyl-phenyl)-1,1-dimethyl-ethylamino]-1-hydroxy-ethyl}-6-hydroxy-4H-benzo[1,4]oxazin-3-on

Die Hydrierung von 0.70 g (1.4 mmol) 6-Benzyloxy-8-{2-[2-(4-fluor-3-methyl-phenyl)-1,1-dimethyl-ethylamino]-1-hydroxy-ethyl}-4H-benzo[1,4]oxazin-3-on liefert die Zielverbindung als weißen Feststoff. Ausbeute: 0.50 g (87%, Hydrochlorid); Smp. = 278-280°C; Massenspektroskopie: [M+H]⁺ = 389.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 9: 8-{2-[2-(4-Fluor-2-methyl-phenyl)-1,1-dimethyl-ethylamino]-1-hydroxyethyl}-6-hydroxy-4H-benzo[1,4]oxazin-3-on

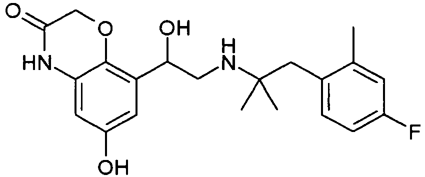

### a) Essigsäure-1-(4-fluor-2-methyl-phenyl)-2-methyl-propyl ester

500 mL einer 0.5 molaren Lösung von 4-Fluor-6-methylphenylmagnesiumbromid und 23.2 mL (260 mmol) Isopropylaldehyd werden analog zu Beispiel 8a) umgesetzt. Nach Hydrolyse mit 10%iger Ammoniumchlorid-Lösung wird die wässrige Phase abgetrennt und mit Diethylether extrahiert. Die vereinigten organischen Phasen werden mit Natriumsulfat getrocknet und eingeengt. Der so erhaltene Alkohol wird dann in 50 mL Acetanhydrid gelöst, mit 1 mL konz. Schwefelsäure versetzt und drei Stunden unter Rückfluß gerührt. Dann wird die Reaktionsmischung auf Wasser gegossen, eine weitere Stunde gerührt und alkalisch gestellt. Man extrahiert mit Dichlormethan, trocknet die organischen Phasen mit Natriumsulfat und destilliert die Lösungsmittel ab. Eine fraktionierte Destillation des Rückstands liefert das Produkt in Form einer farblosen Flüssigkeit (Sdp. 105-110°C/8 mbar). Ausbeute 29.0 g (52%).

### b) N-[2-(4-Fluor-2-methyl-phenyl)-1,1-dimethyl-ethyl]-formamid

29.0 g (130 mmol) Essigsäure-1-(4-fluor-2-methyl-phenyl)-2-methyl-propyl ester werden analog zur Vorschrift für Beispiel 8b) umgesetzt und aufgearbeitet. Gelbes Öl. Ausbeute: 27.0 g (99%).

### c) 2-(4-Fluor-2-methyl-phenyl)-1,1-dimethyl-ethylamin

Zur Herstellung des Amins werden 27.0 g (130 mmol) N-[2-(4-Fluor-2-methyl-phenyl)-1,1-dimethyl-ethyl]-formamid wie in der Vorschrift für Beispiel 8c) beschrieben umgesetzt. Weißer Feststoff. Ausbeute: 15.5 g (55%, Hydrochlorid); Smp. = 277-280°C.

### d) 6-Benzyloxy-8-{2-[2-[4-fluor-2-methyl-phenyl)-1,1-dimethyl-ethylamino]-1-hydroxyethyl}-4H-benzo[1,4]oxazin-3-on

Darstellung in Analogie zur Vorschrift für Beispiel 6d) aus 0.95 g (2.66 mmol) Benzyloxy-8-(2-ethoxy-2-hydroxy-acetyl)-4H-benzo[1,4]oxazin-3-on und 0.43 g (2.37 mmol) 2-(4-Fluor-2-methyl-phenyl)-1,1-dimethyl-ethylamin. Ausbeute: 0.75 g (55%, Hydrochlorid); Smp. = 233-236°C.

### e) 8-{2-[2-(4-Fluor-2-methyl-phenyl)-1,1-dimethyl-ethylamino]-1-hydroxy-ethyl}-6-hydroxy-4H-benzo[1,4]oxazin-3-on

Die Debenzylierung von 0.70 g (1.36 mmol) 6-Benzyloxy-8-{2-[2-[4-fluor-2-methylphenyl)-1,1-dimethyl-ethylamino]-1-hydroxy-ethyl}-4H-benzo[1,4]oxazin-3-on liefert die Zielverbindung in Form eines weißen Feststoffs. Ausbeute: 0.50 g (87%, Hydrochlorid); Smp. = 278-280°C; Massenspektroskopie: [M+H]⁺ = 389.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 10: 8-{2-[2-(2,4-Difluor-phenyl)-1,1-dimethyl-ethylaminol-1-hydroxy-ethyl}-6-hydroxy-4H-benzo[1,4]oxazin-3-on

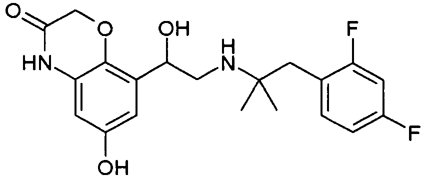

### a) 1-(2,4-Difluor-phenyl)-2-methyl-propan-2-ol

Zu einer Lösung von 500 mL 0.25 molarem 2,4-Difluorbenzylmagnesiumbromid in Diethylether werden innerhalb von 20 Minuten 11.0 mL Aceton, verdünnt mit 50 mL Diethylether, zugetropft. Anschließend wird 1.5 Stunden unter Rückfluß gerührt und dann mit 10%iger Ammoniumchlorid-Lösung hydrolysiert. Die Etherphase wird abgetrennt, mit Wasser gewaschen, mit Natriumsulfat getrocknet und eingeengt. Die fraktionierte Destillation des Rückstands liefert den Alkohol als farblose Flüssigkeit (Sdp. 70-73°C/ 2 mmbar). Ausbeute: 20.0 g (86%).

### b) N-[2-(2,4-Difluor-phenyl]-1,1-dimethyl]-formamid

Ritter-Reaktion mit 20 g (110 mmol) 1-(2,4-Difluor-phenyl)-2-methyl-propan-2-ol nach dem für Beispiel 8b) beschriebenen Verfahren. Gelbes Öl. Ausbeute: 22.0 g (94%).

### c) 2-(2,4-Difluor-phenyl)-1,1-dimethyl-ethylamin

Umsetzung von 22.0 g (100 mmol) N-[2-(2,4-Difluor-phenyl]-1,1-dimethyl-ethyl]-formamid in Analogie zur Vorschrift für Beispiel 8c). Ausbeute: 16.0 g (72%, Hydrochlorid); Smp. = 201-203°C.

### d) 6-Benzyloxy-8-{2-[2-(2,4-difluor-phenyl)-1,1-dimethyl-ethylamino]-1-hydroxy-ethyl}-4H-benzo[1,4]oxazin-3-on

Umsetzung von 0.89 g (2.49 mmol) Benzyloxy-8-(2-ethoxy-2-hydroxy-acetyl)-4H-benzo[1,4]oxazin-3-on und 0.40 g (2.16 mmol) 2-(2,4-Difluor-phenyl)-1,1-dimethylethylamin in der für Beispiel 6d) beschriebenen Weise. Ausbeute: 0.80 g (62%, Hydrochlorid); Smp. = 245-247°C.

### e) 8-{2-[2-(2,4-Difluor-phenyl)-1,1-dimethylamino]-1-hydroxy-ethyl}-6-hydroxy-4H-benzo[1,4]oxazin-3-on

Die Hydrogenolyse von 0.70 g (1.35 mmol) 6-Benzyloxy-8-{2-[2-(2,4-difluor-phenyl)-1,1-dimethyl-ethylamino]-1-hydroxy-ethyl}-4H-benzo[1,4]oxazin-3-on liefert die Zielverbindung als weißen Feststoff. Ausbeute: 0.48 g (83%, Hydrochlorid); Smp. = 279-280°C; Massenspektroskopie: [M+H]⁺ = 393.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 11: 8-{2-[2-(3,5-Difluor-phenyl)-1,1-dimethyl-ethylamino]-1-hydroxy-ethyl}-6-hydroxy-4H-benzo[1,4]oxazin-3-on

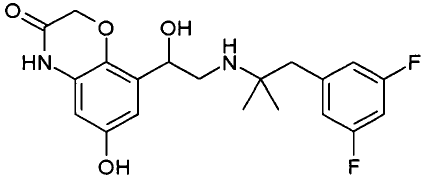

### a) 1-(3,5-Difluor-phenyl)-2-methyl-propan-2-ol

Die Zielverbindung wird durch Umsetzung einer Grignardverbindung, hergestellt aus 25.0 g (121 mmol) 3,5-Difluorbenzylbromid, mit 12.6 mL (171 mmol) Aceton erhalten. Gelbes Öl. Ausbeute: 13.5 g (60%).

### b) 2-(3,5-Difluor-phenyl)-1,1-dimethyl-ethylamin

Die Ritter-Reaktion von 5.5 g (29.5 mmol) 1-(3,5-Difluor-phenyl)-2-methyl-propan-2-ol und 1.8 g Natriumcyanid liefert 7.0 g Formamid, das zur Abspaltung der Formylgruppe mit Salzsäure behandelt wird. Leicht gelbes Öl. Ausbeute: 4.6 g (75%).

### c) 6-Benzyloxy-8-{2-[2-(3,5-difluor-phenyl)-1,1-dimethyl-ethylamino]-1-hydroxy-ethyl}-4H-benzo[1,4]oxazin-3-on

Darstellung aus 1.73 g (4.84 mmol) Benzyloxy-8-(2-ethoxy-2-hydroxy-acetyl)-4H-benzo[1,4]oxazin-3-on und 0.80 g (4.32 mmol) 2-(3,5-Difluor-phenyl)-1,1-dimethylethylamin in der üblichen Weise. Ausbeute: 1.50 g (58 %, Hydrochlorid); Smp. = 240-244°C.

### d) 8-{2-[2-(3,5-Difluor-phenyl)-1,1-dimethyl-ethylamino]-1-hydroxy-ethyl}-6-hydroxy-4H-benzo[1,4]oxazin-3-on

Hydrogenolyse von 1.30 g (2.43 mmol) 6-Benzyloxy-8-{2-[2-(3,5-difluor-phenyl)-1,1-dimethyl-ethylamino]-1-hydroxy-ethyl}-4H-benzo[1,4]oxazin-3-on liefert die Zielverbindung als weißen Feststoff. Ausbeute: 0.90 g (86%, Hydrochlorid); Smp. = 150-158°C; Massenspektroskopie: [M+H]⁺ = 393.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 12: 8-{2-[2-(4-Ethoxy-phenyl)-1,1-dimethyl-ethylamino]-1-hydroxy-ethyl}-6-hydroxy-4H-benzo[1,4]oxazin-3-on

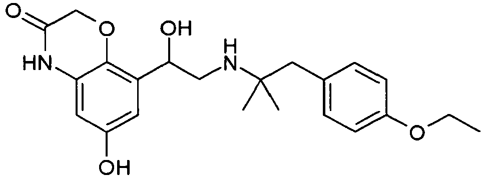

### a) [2-(4-Ethoxy-phenyl)-1,1-dimethyl-ethyl]-carbaminsäure benzyl ester

15.0 g (50 mmol) [2-(4-Hydroxy-phenyl)-1,1-dimethyl-ethyl]-carbaminsäure benzyl ester werden mit 7.5 mL (92 mmol) Ethyliodid und 21 g (150 mmol) Kaliumcarbonat über 10 Stunden bei 90-100°C gerührt. Die Reaktionsmischung wird mit Ethylacetat versetzt, zweimal mit Wasser gewaschen und mit Natriumsulfat getrocknet. Nach dem Abdestillieren der Lösungsmittel bleibt ein gelbes Öl (15.0 g, 92%) zurück, das direkt weiter umgesetzt wird.

### b) 2-(4-Ethoxy-phenyl)-1,1-dimethyl-ethylamin

Eine Lösung von 15.0 g (49 mmol) [2-(4-Ethoxy-phenyl)-1,1-dimethyl-ethyl]-carbaminsäure benzyl ester in 100 mL Eisessig wird mit 2 g Palladium auf Kohle (10%ig) versetzt und anschließend bei 5 bar und 40 bis 50°C hydriert. Der Katalysator wird abfiltriert und das Filtrat vom Lösungsmittel befreit. Der Rückstand wird in wenig Wasser gelöst, mit konz. Natronlauge alkalisch gestellt und mit Ethylacetat extrahiert. Die organische Phase wird mit Wasser gewaschen, mit Natriumsulfat getrocknet und eingeengt. Das Rohprodukt wird in Acetonitril gelöst und mit etherischer Salzsäure angesäuert. Der nach Zugabe von Diethylether ausfallende Feststoff wird abgesaugt und getrocknet. Ausbeute: 8.8 g (Hydrochlorid, 84%); Smp. = 198-200°C.

### c) 6-Benzyloxy-8-{2-[2-(4-ethoxy-phenyl)-1,1-dimethyl-ethylamino]-1-hydroxy-ethyl}-4H-benzo[1,4]oxazin-3-on

2.14 g (6.0 mmol) 6-Benzyloxy-8-(2-ethoxy-2-hydroxy-acetyl)-4H-benzo[1,4]oxazin-3-on und 1.0 g (5.2 mmol) 2-(4-Ethoxy-phenyl)-1,1-dimethyl-ethylamin werden in 40 mL Ethanol eine Stunde bei 50-80°C gerührt. Nach Abkühlung auf Raumtemperatur werden 0.23 g (6.0 mmol) Natriumborhydrid zugegeben und man läßt über eine weitere Stunde rühren. Die Reaktionsmischung wird mit 5 ml Aceton versetzt, 30 Minuten gerührt, mit Eisessig angesäuert und eingeengt. Der Rückstand wird mit Wasser und Ethylacetat versetzt und alkalisch gestellt. Die organische Phase wird abgetrennt, mit Wasser gewaschen, mit Natriumsulfat getrocknet und im Vakuum vom Lösungsmittel befreit. Der Rückstand wird wieder in Ethylacetat und Wasser gelöst, mit konz. Salzsäure versetzt und mit Diethylether verdünnt. Der ausfallende Feststoff wird abgesaugt und mit Diethylether gewaschen. Weißer Feststoff. Ausbeute: 2.0 g (61 %, Hydrochlorid); Smp. = 214-216°C.

### d) 8-{2-[2-(4-Ethoxy-phenyl)-1,1-dimethyl-ethylamino]-1-hydroxy-ethyl}-6-hydroxy-4H-benzo[1,4]oxazin-3-on

1.5 g (2.8 mmol) 6-Benzyloxy-8-{2-[2-(4-ethoxy-phenyl)-1,1-dimethyl-ethylamino]-1-hydroxy-ethyl}-4H-benzo[1,4]oxazin-3-on in 80 mL Methanol werden mit 250 mg Palladium auf Kohle (10 %ig) als Katalysator bei Raumtemperatur und Normaldruck hydriert. Der Katalysator wird abgesaugt und das Filtrat eingeengt. Der Rückstand wird in 5 mL Ethanol durch Erwärmen gelöst, angeimpft und mit Ethylacetat verdünnt. Der ausfallende Feststoff wird abfiltriert und gewaschen. Weißer Feststoff. Ausbeute 1.0 g (83%, Hydrochlorid); Smp= 232-235°C; Massenspektrometrie: [M+H]⁺ = 401.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 13: 8-{2-[2-(3,5-Dimethyl-phenyl)-1,1-dimethyl-ethylamino]-1-hydroxy-ethyl}-6-hydroxy-4H-benzo[1,4]oxazin-3-on

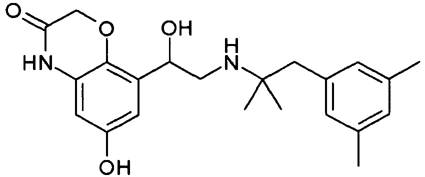

### a) 1-(3,5-Dimethyl-phenyl)-2-methyl-propanol-2-ol

Erhalten aus der Umsetzung von (3,5-Dimethyl-phenyl)-essigsäureethylester mit Methylmagnesiumbromid.

### b) 2-(3,5-Dimethyl-phenyl)-1,1-dimethyl-ethylamin

Durch Umsetzung von 6.00 g (34 mmol) 1-(3,5-Dimethyl-phenyl)-2-methyl-propanol-2-ol und 2.00 g (41 mmol) Natriumcyanid in einer Ritter-Reaktion werden 2.40 g 2-(3,5-Dimethyl-phenyl)-1,1-dimethyl-ethylformamid (35% Ausbeute) erhalten. Zur Freisetzung des Amins wird das Formamid (2.40 g, 11.7 mmol) mit Salzsäure behandelt. Die Durchführung und Aufarbeitung erfolgen in Analogie zur Vorschrift für Beispiel 8c). Öl. Ausbeute: 1.70 g (82%); Massenspektroskopie: [M+H]⁺ = 178.

### c) 6-Benzyloxy-8-{2-[2-(3,5-dimethyl-phenyl)-1,1-dimethyl-ethylamino]-1-hydroxyethyl}-4H-benzo[1,4]oxazin-3-on

Darstellung in Analogie zur Vorschrift für Beispiel 8d) aus 1.47 g (4.1 mmol) Benzyloxy-8-(2-ethoxy-2-hydroxy-acetyl)-4H-benzo[1,4]oxazin-3-on und 0.65 g (3.7 mmol) 2-(3,5-Dimethyl-phenyl)-1,1-dimethyl-ethylamin. Ausbeute: 1.1 g (51%, Hydrochlorid); Smp. = 220-222°C.

### d) 8-{2-[2-(3,5-Dimethyl-phenyl)-1,1-dimethyl-ethylamino]-1-hydroxy-ethyl}-6-hydroxy-4H-benzo[1,4]oxazin-3-on

Die Zielverbindung wurde nach Hydrogenolyse von 0.90 g (1.71 mmol) 6-Benzyloxy-8-{2-[2-(3,5-dimethyl-phenyl)-1,1-dimethyl-ethylamino]-1-hydroxy-ethyl}-4H-benzo[1,4]oxazin-3-on und Umkristallisation des Rohproduktes aus Isopropanol erhalten. Weißer Feststoff. Ausbeute: 0.50 g (69%, Hydrochlorid); Smp. = 235-238°C; Massenspektroskopie: [M+H]⁺ = 385.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 14: 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure

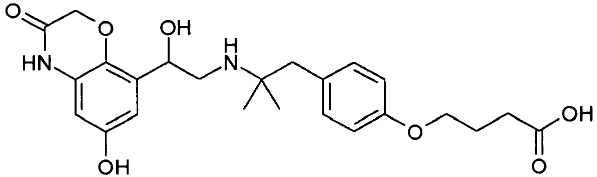

### a) 4-[4-(2-Amino-2-methyl-propyl)-phenoxyl-buttersäureethylester

4.5 g (15.0 mmol) [2-(4-Hydroxy-phenyl)-1,1-dimethyl-ethyl]-carbaminsäure benzyl ester, 2.3 mL (16.0 mmol) 4-Brom-buttersäureethylester, 2.3 g (16.6 mmol) Kaliumcarbonat und 0.3 g (1.8 mmol) Kaliumiodid in 20 mL Dimethylformamid werden 13 Stunden bei 120°C erwärmt. Die Reaktionsmischung wird mit Ethylacetat verdünnt und nacheinander mit Wasser, Natriumhydroxid-Lösung und Wasser gewaschen. Die organische Phase wird mit Natriumsulfat getrocknet und eingeengt. Den Rückstand reinigt man chromatographisch (Laufmittel: Cyclohexan/Ethylacetat = 9:1). Es werden 5.0 g eines gelben Öls isoliert, das in 50 mL Essigsäure gelöst wird und mit 1.0 g Palladium auf Kohle als Katalysator bei 40°C und 3 bar hydriert wird. Der Kataysator wird abfiltriert und das Filtrat vom Lösungsmitttel befreit. Der Rückstand wird in Diethylether gelöst und mit etherischer Salzsäure versetzt. Der ausfallende Feststoff wird abgesaugt und getrocknet. Ausbeute: 2.9 g (66% über zwei Stufen, Hydrochlorid); Smp. = 103-105°C.

### b) 4-(4-{2-[2-(6-Benzyloxy-3-oxo-3,4-dihydro-2H-benzo[1,4]oxazin-8-yl)-2-hydroxy-ethylamino]-2-methyl-propyl}-phenoxy)-buttersäureethylester

1.20 g (3.36 mmol) Benzyloxy-8-(2-ethoxy-2-hydroxy-acetyl)-4H-benzo[1,4]oxazin-3-on und 0.90 g (3.22 mmol) 4-[4-(2-Amino-2-methyl-propyl)-phenoxy]-buttersäureethylester werden in der für Beispiel 8d) beschriebenen Weise umgesetzt. Das Rohprodukt wird in 10 mL Ethylacetat und 10 mL Wasser gelöst und unter Rühren mit Oxalsäure versetzt. Die Lösung verdünnt man mit Diethylether und der ausfallende Feststoff wird abgesaugt und mit Diethylether gewaschen. Ausbeute: 1.20 g (54%, Oxalat); Smp. = 223-227°C.

### c) 4-(4-{2-[2-(6-Benzyloxy-3-oxo-3,4-dihydro-2H-benzo[1,4]oxazin-8-yl)-2-hydroxy-ethylamino]-2-methyl-propyl}-phenoxy)-buttersäure

Eine Lösung von 1.00 g (1.73 mmol) 4-(4-{2-[2-(6-Benzyloxy-3-oxo-3,4-dihydro-2H-benzo[1,4]oxazin-8-yl)-2-hydroxy-ethylamino]-2-methyl-propyl}-phenoxy)-buttersäureethylester in 25 mL Methanol wird mit 2.5 mL 1 N Natriumhydroxid-Lösung versetzt, 30 Minuten refluxiert und dann mit 1 N Salzsäure neutralisiert. Die Lösung wird eingeengt und das zurückbleibende Öl durch Erwärmen in 5 mL n-Butanol gelöst. Nach Zugabe einer Kristallisationshilfe fällt ein Feststoff aus, der abgesaugt und mit Aceton und Diethylether gewaschen wird. Ausbeute: 0.75 g (79%); Smp. = 216-218°C.

### d) 4-(4-{2-[2-Hydroxy-(6-hydroxy-3-oxo-3,4-dihydro-2H-benzo[1,4]oxazin-8-yl)-ethylamino]-2-methyl-propyl}-phenoxy)-buttersäure

0.70 g (1.28 mmol) 4-(4-{2-[2-(6-Benzyloxy-3-oxo-3,4-dihydro-2H-benzo[1,4]oxazin-8-yl)-2-hydroxy-ethylamino]-2-methyl-propyl}-phenoxy)-buttersäure werden in 25 mL Methanol und 2 mL Essigsäure gelöst und in Gegenwart von 150 mg Palladium auf Kohle (10%ig) bei Raumtemperatur und Normaldruck hydriert. Der Katalysator wird abfiltriert und das Filtrat vom Lösungsmittel befreit. Das Produkt erhält man durch Kristallisation aus einem Methanol/Aceton-Gemisch. Ausbeute: 0.40 g (68%); Smp. = 201-204°C; Massenspektroskopie: [M+H]⁺ = 459.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 15: 8-{2-[2-(3,4-Difluor-phenyl)-1,1-dimethyl-ethylaminol-1-hydroxy-ethyl}-6-hydroxy-4H-benzo[1,4]oxazin-3-on

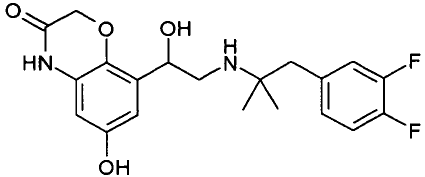

### a) 1-(3,4-Difluor-phenyl)-2-methyl-propan-2-ol

Aus 23.0 g (111 mmol) 3,4-Difluorbenzylbromid wird eine Grignardverbindung hergestellt, die man dann mit 11.6 mL (158 mmol) Aceton umsetzt. Leicht gelbes Öl. Ausbeute: 9.7 g (47%); R_{f}-Wert: 0.55 (Ethylacetat/Petrolether = 1:3).

### b) N-[2-(3,4-Difluor-phenyl)-1,1-dimethyl-ethyl]-formamid

Die Zielverbindung wird über eine Ritter-Reaktion mit 4.0 g (21.5 mmol) 1-(3,4-Difluorphenyl)-2-methyl-propan-2-ol erhalten. Leicht gelbes Öl. Ausbeute: 4.0 g (87%); Massenspektrometrie: [M+H]⁺ = 214.

### c) 2-(3,4-Difluor-phenyl)-1,1-dimethyl-ethylamin

4.00 g (18.5 mmol) N-[2-(3,4-Difluor-phenyl)-1,1-dimethyl-ethyl]-formamid werden in Ethanol gelöst, mit konz. Salzsäure versetzt und über Nacht unter Rückfluß erwärmt. Die Reaktionslösung wird auf Eiswasser gegossen, mit Natriumhydroxid alkalisch gestellt und mit tert-Butylmethylether extrahiert. Die organischen Phasen werden mit Wasser gewaschen, mit Natriumsulfat getrocknet und eingeengt. Gelbes Öl. Ausbeute: 3.2 g (92%); Massenspektrometrie: [M+H]⁺ = 186.

### d) 8-{2-[2-(3,4-Difluor-phenyl)-1,1-dimethyl-ethylamino]-1-hydroxy-ethyl}-6-hydroxy-4H-benzo[1,4]oxazin-3-on

357 mg (1 mmol) 6-Benzyloxy-8-(2-ethoxy-2-hydroxy-acetyl)-4H-benzo[1,4]oxazin-3-on und 185 mg (1 mmol) 2-(3,4-Difluor-phenyl)-1,1-dimethyl-ethylamin werden 30 Minuten in 5 mL Tetrahydrofuran bei Raumtemperatur gerührt. Man kühlt auf 0°C ab und tropft unter Argonatmosphäre 1.5 mL einer 2 molaren Lösung von Lithiumborhydrid in Tetrahydrofuran zu. Es wird 30 min bei Raumtemperatur gerührt, mit 10 mL Dichlormethan und 3 mL Wasser versetzt, eine weitere Stunde gerührt und dann über Extrelut ^{®} filtriert. Das das Ethanolamin enthaltende Eluat wird vom Lösungsmittel befreit. Der Rückstand wird in Methanol gelöst und mit Palladium auf Kohle (10%ig) als Katalysator bei 2.5 bar und Raumtemperatur hydriert. Anschließend wird der Katalysator

abgetrennt und das Rohprodukt chromatographisch gereinigt. Weißer Feststoff. Ausbeute: 31 mg (6%, Trifluorethylacetat); Massenspektroskopie: [M+H]⁺ = 393.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 16: 8-{2-[2-(2-Chlor-4-fluor-phenyl)-1,1-dimethyl-ethylamino]-1-hydroxyethyl}-6-hydroxy-4H-benzo[1,4]oxazin-3-on

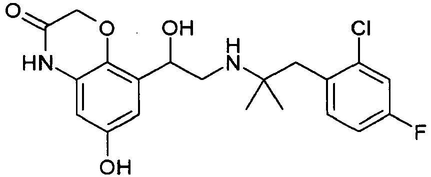

### a) 1-(2-Chlor-4-fluor-phenyl)-2-methyl-propan-2-ol

Herstellung aus 20 g (97 mmol) (2-Chlor-4-fluor-phenyl)-essigsäuremethylester und 98 mL einer 3 molaren Lösung von Methylmagnesiumbromid in Analogie zur Vorschrift für Beispiel 6a).

### b) N-[2-(2-Chlor-4-fluor-phenyl)-1,1-dimethyl-ethyl]-formamid

7.5 g (37 mmol) 1-(2-Chlor-4-fluor-phenyl)-2-methyl-propan-2-ol wurden nach der für Beispiel 8b) beschriebenen Vorschrift umgesetzt und aufgearbeitet. Das so erhaltene Öl wurde zur weiteren Reinigung an einer kurzen Kieselgelsäule chromatographiert (Petrolether/Ethylacetat = 9:1). Öl. Ausbeute 7.4 g (87%); Massenspektrometrie: [M+H]⁺ = 230/232.

### c) 2-(2-Chlor-4-fluor-phenyl)-1,1-dimethyl-ethylamin

Umsetzung von 7.4 g (32 mmol) N-[2-(2-Chlor-4-fluor-phenyl)-1,1-dimethyl-ethyl]-formamid wie in der Vorschrift für Beispiel 15c) beschrieben. Braunes Öl. Ausbeute: 5.14 g (79%); Massenspektrometrie: [M+H]⁺ = 202/204.

### d) 8-{2-[2-(2-Chlor-4-fluor-phenyl)-1,1-dimethyl-ethylamino]-1-hydroxy-ethyl}-6-hydroxy-4H-benzo[1,4]oxazin-3-on

357 mg (1 mmol) 6-Benzyloxy-8-(2-ethoxy-2-hydroxy-acetyl)-4H-benzo[1,4]oxazin-3-on und 202 mg (1 mmol) 2-(2-Chlor-4-fluor-phenyl)-1,1-dimethyl-ethylamin werden in Analogie zur Vorschrift für Beispiel 8d) mit Lithiumborhydrid umgesetzt. Zur Debenzylierung des so erhaltenen Ethanolamins wird dieses in 3 mL Dichlormethan gelöst und auf -78°C abgekühlt. Bei dieser Temperatur tropft man 2 ml einer 1 molaren Lösung von Bortribromid in Dichlormethan zu und läßt langsam auf Raumtemperatur erwärmen. Die Reaktionsmischung wird mit 10 mL Dichlormethan und 3 mL Wasser versetzt und über Extrelut^{®} filtriert. Das Eluat wird vom Lösungsmittel befreit und der Rückstand mittels Chromatographie gereinigt. Weißer Feststoff. Ausbeute: 70 mg (13%, Trifluorethylacetat); Massenspektroskopie: [M+H]⁺ = 409/11.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 17: 8-{2-[2-(4-chlor-phenyl)-1,1-dimethyl-ethylamino]-1-hydroxy-ethyl}-6-hydroxy-4H-benzo[1,4]oxazin-3-on

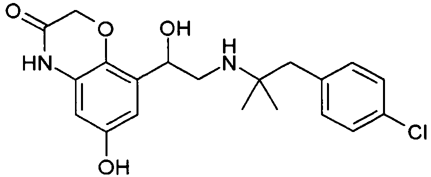

Eine Lösung von 300 mg (0.91 mmol) 6-Benzyloxy-8-(2,2-dihydroxy-acetyl)-4H-benzo[1,4]oxazin-3-on und 200 mg (1.09 mmol) 2-(4-Chlor-phenyl)-1,1-dimethylethylamin in 3 mL Ethanol wurde mit Molsieb versetzt und 90 Minuten bei 80°C gerührt. Man ließ auf Raumtemperatur abkühlen, fügte 35 mg (0.91 mmol) Natriumborhydrid hinzu und ließ 1 Stunde rühren. Anschließend wurde die Reaktionsmischung mit Natriumhydrogencarbonat-Lösung versetzt und mit Ethylacetat extrahiert. Die vereinigten organischen Phasen wurden vom Lösungsmittel befreit und der Rückstand chromatographiert (Laufmittel: Hexan/Ethylacetat/Methanol), wobei 305 mg Ethanolamin gewonnen werden konnten. Dieses wurde in 3 mL Dichlormethan gelöst und unter Argonatmosphäre auf -78°C abgekühlt. Es wurden 3 mL einer 1 molaren Lösung von Bortribromid in Dichlormethan zugetropft und man ließ eine Stunde bei -78°C und 20 Minuten bei Raumtemperatur Rühren. Dann tropfte man bei -78°C 3 mL konz. AmmoniakLösung zu und ließ 5 Minuten rühren. Die Reaktionsmischung wurde mit Ammoniumchlorid-Lösung versetzt und mit Ethylacetat extrahiert. Die vereinigten organischen Phasen wurden eingeengt und der Rückstand zur weiteren Reinigung chromatographiert (Kieselgel; Laufmittel: Dichlormethan/Methanol + 1% Ammoniak). Beigefarbener Feststoff: 93 mg (26%); Massenspektrometrie: [M+H]⁺ = 391.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 18: 8-{2-[2-(4-Brom-phenyl)-1,1-dimethyl-ethylamino]-1-hydroxy-ethyl}-6-hydroxy-4H-benzo[1,4]oxazin-3-on

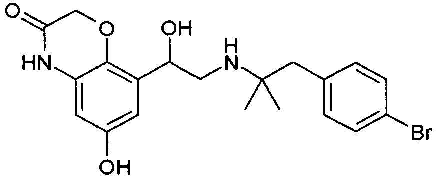

Die Ethanolamindarstellung und Debenzylierung erfolgten in der für Beispiel 17 beschriebenen Weise aus 300 mg (0.91 mmol) 6-Benzyloxy-8-(2,2-dihydroxy-acetyl)-4H-benzo[1,4]oxazin-3-on und 250 mg (1.09) mmol) 2-(4-Brom-phenyl)-1,1-dimethylethylamin. Beigefarbener Feststoff. Ausbeute: 54 mg (14%); Massenspektrometrie: [M+H]⁺ = 435, 437.

Die (R)- und (S)-Enantiomere dieses Ausführungsbeispiels können durch Auftrennung des Racemats in Analogie zu gängigen, im Stand der Technik bekannten Methoden erhalten werden.

### Beispiel 19: 8-{2-[2-(4-Fluor-phenyl)-1,1-dimethyl-ethylamino]-1-hydroxy-ethyl}-6-hydroxy-4H-benzo[1,4]oxazin-3-on

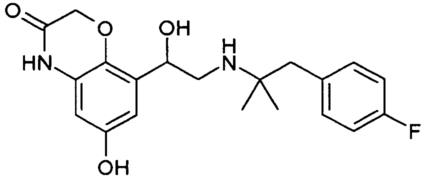

300 mg (0.91 mmol) 6-Benzyloxy-8-(2,2-dihydroxy-acetyl)-4H-benzo[1,4]oxazin-3-on und 183 mg (1.09 mmol) 2-(4-Fluor-phenyl)-1,1-dimethyl-ethylamin wurden in 3 ml Ethanol gelöst. Es wurde Molsieb zugegeben und 30 Minuten auf 80°C erwärmt. Nach Abkühlung auf Raumtemperatur wurden 35 mg (0.91 mmol) Natriumborhydrid zugegeben. Man ließ 1 Stunde bei Raumtemperatur rühren, gab dann zur Reaktionsmischung Natriumhydrogencarbonat-Lösung und extrahierte mit Ethylacetat. Die organischen Phasen wurden eingeengt und der Rückstand chromatograhiert (Laufmittel: Hexan/Ethylacetat/Methanol). Das so erhaltene Ethanolamin (223 mg) wurde zur Abspaltung der Benzylschutzgruppe in Methanol gelöst und mit 150 mg Palladiumhydroxid als Katalysator bei Raumtemperatur und Normaldruck hydriert. Der Katalysator wurde mittels Filtration über Celite® abgetrennt, das Filtrat vom Lösungsmittel befreit und der Rückstand chromatographiert (Kieselgel; Laufmittel: Dichlormethan/Methanol). Beigefarbener Feststoff. Ausbeute: 76 mg (22%); Massenspektrometrie: [M+H]⁺ = 375.

Die (R)- und (S)-Enantiomere dieses Ausführungsbeispiels können durch Auftrennung des Racemats in Analogie zu gängigen, im Stand der Technik bekannten Methoden erhalten werden.

In Analogie zu den vorstehend beschriebenen Synthesebeispielen können ferner die nachfolgenden, erfindungsgemäßen Verbindungen der Formel **1** erhalten werden:
**Beispiel 20:** 8-{2-[2-(4-Fluor-3-methoxy-phenyl)-1,1-dimethyl-ethylamino]-1-hydroxyethyl}-6-hydroxy-4H-benzo[1,4]oxazin-3-on;
**Beispiel 21:** 8-{2-[2-(4-Fluor-2,6-dimethyl-phenyl)-1,1-dimethyl-ethylamino]-1-hydroxyethyl}-6-hydroxy-4H-benzo[1,4]oxazin-3-on;
**Beispiel 22:** 8-{2-[2-(4-Chlor-2-methyl-phenyl)-1,1-dimethyl-ethylamino]-1-hydroxyethyl}-6-hydroxy-4H-benzo[1,4]oxazin-3-on;
**Beispiel 23:** 8-{2-[2-(4-Chlor-3-fluor-phenyl)-1,1-dimethyl-ethylamino]-1-hydroxyethyl}-6-hydroxy-4H-benzo[1,4]oxazin-3-on;
**Beispiel 24:** 8-{2-[2-(4-Chlor-2-fluor-phenyl)-1,1-dimethyl-ethylamino]-1-hydroxyethyl}-6-hydroxy-4H-benzo[1,4]oxazin-3-on;
**Beispiel 25:** 8-{2-[2-(3-Chlor-4-fluor-phenyl)-1,1-dimethyl-ethylamino]-1-hydroxyethyl }-6-hydroxy-4H-benzo[1,4]oxazin-3-on;
**Beispiel 26:** 8-{2-[2-(2,6-Difluor-4-methoxy-phenyl)-1,1-dimethyl-ethylamino]-1-hydroxy-ethyl }-6-hydroxy-4H-benzo[1,4]oxazin-3-on;
**Beispiel 27:** 8-{2-[2-(2,5-Difluor-4-methoxy-phenyl)-1,1-dimethyl-ethylamino]-1-hydroxy-ethyl }-6-hydroxy-4H-benzo[1,4]oxazin-3 -on;
**Beispiel 28:** 8-{2-[2-(4-Fluor-3,5-dimethyl-phenyl)-1,1-dimethyl-ethylamino]-1-hydroxyethyl}-6-hydroxy-4H-benzo[1,4]oxazin-3-on;
**Beispiel 29:** 8-{2-[2-(3,5-Dichlor-phenyl)-1,1-dimethyl-ethylamino]-1-hydroxy-ethyl}-6-hydroxy-4H-benzo[1,4]oxazin-3-on;
**Beispiel 30:** 8-{2-[2-(4-Chlor-3-methyl-phenyl)-1,1-dimethyl-ethylamino]-1-hydroxyethyl}-6-hydroxy-4H-benzo[1,4]oxazin-3-on;
**Beispiel 31:** 8-{2-[2-(3,4,5-Trifluor-phenyl)-1,1-dimethyl-ethylamino]-1-hydroxy-ethyl}-6-hydroxy-4H-benzo[1,4]oxazin-3-on;
**Beispiel 32:** 8-{2-[2-(3-Methyl-phenyl)-1,1-dimethyl-ethylamino]-1-hydroxy-ethyl}-6-hydroxy-4H-benzo[1,4]oxazin-3-on;
**Beispiel 33:** 8- {2-[2-(3,4-Dichlor-phenyl)-1,1-dimethyl-ethylamino]-1-hydroxy-ethyl}-6-hydroxy-4H-benzo[1,4]oxazin-3-on.

Geeignete Anwendungsformen zur Applikation der Verbindungen der Formel 1 sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können. Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilflösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäßen Applikation der Verbindungen der Formel **1** zur Therapie von Asthma werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten. Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.

Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und

Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.

Den treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester..Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und dem Wirkstoff **1** nur noch Benzalkoniumchlorid und Natriumedetat.

In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen.

Ein weiterer Aspekt der vorliegenden Erfindung betrifft die vorstehend genannten pharmazeutischen Formulierungen, gekennzeichnet durch einen Gehalt einer Verbindung der Formel **1**, als solche, besonders bevorzugt die vorstehend genannten inhalativ applizierbaren pharmazeutischen Formulierungen.

Die nachfolgenden Formulierungsbeipiele illustrieren die vorliegende Erfindung, ohne sie jedoch in ihrem Umfang zu beschränken:

### Pharmazeutische Formulierungsbeispiele

| A) | Tabletten | pro Tablette |
|---|---|---|
| | Wirkstoff **1** | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff 1 | 80 mg |
| | Milchzucker | 55 mg |
| | Maisstärke | 190 mg |
| | Mikrokristalline Cellulose | 3 5 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff **1** | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | Wirkstoff **1** | 0,005 |
| | Sorbitantrioleat | 0,1 |
| | Monofluortrichlormethan und TG134a: TG227 2:1 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | Wirkstoff **1** | 333.3 mg |
| | Benzalkoniumchlorid | 10.0 mg |
| | EDTA | 50.0 mg |
| | HCl (1n) | ad pH 3.4 |

Diese Lösung kann in üblicher Art und Weise hergestellt werden.

| F) | Inhalationpulver | |
|---|---|---|
| | Wirkstoff **1** | 12 µg |
| | Lactose Monohydrat | ad 25 mg |

Die Herstellung des Inhalationspulvers erfolgt in üblicher Art und Weise durch Mischen der einzelnen Bestandteile.

## Patentansprüche

1. Verwendung von Verbindungen der allgemeinen Formel **1** 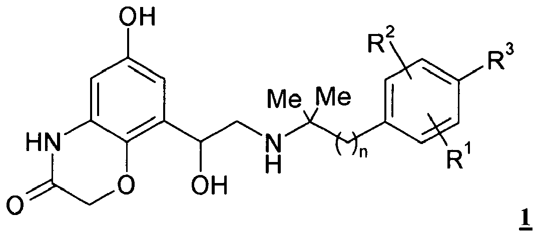 worin
n 1;
R¹ Wasserstoff, Halogen, C₁-C₄-Alkyl oder -O-C₁-C₄-Alkyl;
R² Wasserstoff, Halogen, C₁-C₄-Alkyl oder -O-C₁-C₄-Alkyl;
R³ C₁-C₄-Alkyl, OH, Halogen, -O-C₁-C₄-Alkyl, -O-C₁-C₄-Alkylen-COOH, -O-C₁-C₄-Alkylen-CO-O-C₁-C₄-Alkyl,
bedeuten, mit der Maßgabe, daß wenn R¹ und R² jeweils ortho-Methyl bedeuten, R³ nicht gleichzeitig OH sein kann, zur Herstellung eines Arzneimittels zur Behandlung von Atemwegserkrankungen, die ausgewählt sind aus der Gruppe bestehend aus Asthma Bronchiale, pädiatrisches Asthma, schweres Asthma, akuter Asthma-Anfall, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

2. Verwendung von Verbindungen der allgemeinen Formel **1** nach Anspruch 1, worin
n 1;
R¹ Wasserstoff, Fluor, Chlor, Methyl oder Methoxy;
R² Wasserstoff, Fluor, Chlor, Methyl oder Methoxy;
R³ C₁-C₄-Alkyl, OH, Fluor, Chlor, Brom, -O-C₁-C₄-Alkyl, -O-C₁-C₄-Alkylen-COOH, -O-C₁-C₄-Alkylen-CO-O-C₁-C₄-Alkyl,
bedeuten, mit der Maßgabe, daß wenn R¹ und R² jeweils ortho-Methyl bedeuten, R³ nicht gleichzeitig OH sein kann.

3. Verwendung von Verbindungen der allgemeinen Formel **1** nach Anspruch 1, worin
n 1;
R¹ Wasserstoff oder C₁-C₄-Alkyl ;
R² Wasserstoff oder C₁-C₄-Alkyl;
R³ C₁-C₄-Alkyl, OH, -O-C₁-C₄-Alkyl, -O-C₁-C₄-Alkylen-COOH oder -O-C₁-C₄-Alkylen-CO-O-C₁-C₄-Alkyl,
bedeuten, mit der Maßgabe, daß wenn R¹ und R² jeweils ortho-Methyl bedeuten, R³ nicht gleichzeitig OH sein kann.

4. Verwendung von Verbindungen der allgemeinen Forrnel **1** nach Anspruch 1, worin
n 1;
R¹ Wasserstoff, Methyl oder Ethyl;
R² Wasserstoff, Methyl oder Ethyl;
R³ Methyl, Ethyl, OH, Methoxy, Ethoxy, -O-CH₂-COOH, -O-CH₂-COOMethyl oder -O-CH₂-COOEthyl,
bedeuten, mit der Maßgabe, daß wenn R¹ und R² jeweils ortho-Methyl bedeuten, R³ nicht gleichzeitig OH sein kann.

5. Verwendung von Verbindungen der allgemeinen Formel **1** nach einem der Ansprüche 1 bis 4, worin
R³ Methoxy, Ethoxy, -O-CH₂-COOH, -O-CH₂-COOMethyl oder -O-CH₂-COOEthyl,
bedeutet und R¹, R² und n die in den Ansprüchen 8 bis 11 genannten Bedeutungen haben können.

6. Verwendung von Verbindungen der allgemeinen Formel **1** nach Anspruch 1, worin
n 1;
R¹ Halogen, C₁-C₄-Alkyl oder -O-C₁-C₄-Alkyl;
R² Halogen, C₁-C₄-Alkyl oder -O-C₁-C₄-Alkyl;
R³ Halogen, C₁-C₄-Alkyl oder -O-C₁-C₄-Alkyl, bedeuten.

7. Verwendung von Verbindungen der allgemeinen Formel **1** nach Anspruch 6, worin
n 1;
R¹ Fluor, Chlor, Methyl oder Methoxy;
R² Fluor, Chlor, Methyl oder Methoxy ;
R³ Fluor, Chlor, Methyl oder Methoxy bedeuten.

8. Verwendung von Verbindungen der allgemeinen Formel **1** nach Anspruch 1, worin
n 1;
R¹ Wasserstoff;
R² Wasserstoff, Fluor, Chlor oder Methyl;
R³ Methyl, Ethyl, iso-Propyl, tert.-Butyl, OH, Fluor, Chlor, Brom, Methoxy, Ethoxy, -O-CH₂-COOH, -O-CH₂-CH₂-COOH, -O-CH₂-CH₂-CH₂-COOH, -O-CH₂-COOMethyl, -O-CH₂-COOEthyl, -O-CH₂-CH₂-COOMethyl, -O-CH₂-CH₂-COOEthyl, -O-CH₂-CH₂-CH₂-COOMethyl, -O-CH₂-CH₂-CH₂-COOEthyl, bedeuten.

9. Verwendung von Verbindungen der allgemeinen Formel **1** nach Anspruch 8, worin
n 1;
R¹ Wasserstoff;
R² Wasserstoff, Fluor, Chlor oder Methyl;
R³ OH, Fluor, Chlor, Methyl, Methoxy, Ethoxy oder -O-CH₂-COOH, bedeuten.

10. Verwendung von Verbindungen der allgemeinen Formel **1** nach Anspruch 1, worin
n 1;
R¹ und R² Wasserstoff;
R³ Methyl, Ethyl, iso-Propyl, tert.-Butyl, OH, Fluor, Chlor, Brom, Methoxy, Ethoxy, -O-CH₂-COOH, -O-CH₂-CH₂-COOH, -O-CH₂-CH₂-CH₂-COOH, -O-CH₂-COOMethyl, -O-CH₂-COOEthyl, -O-CH₂-CH₂-COOMethyl, -O-CH₂-CH₂-COOEthyl, -O-CH₂-CH₂-CH₂-COOMethyl, -O-CH₂-CH₂-CH₂-COOEthyl, bedeuten.

11. Verwendung von Verbindungen der allgemeinen Formel **1** nach Anspruch 10, worin
n 1;
R¹ und R² Wasserstoff;
R³ OH, Fluor, Chlor, Methoxy, Ethoxy,-O-CH₂-COOH, bevorzugt OH, Fluor, Chlor, Ethoxy oder Methoxy, bedeuten.

12. Verwendung von Verbindungen der allgemeinen Formel **1** nach Anspruch 1, worin
n 1;
R¹ Wasserstoff, Halogen, C₁-C₄-Alkyl oder -O-C₁-C₄-Alkyl;
R² Wasserstoff, Halogen, C₁-C₄-Alkyl oder -O-C₁-C₄-Alkyl;
R³ Wasserstoff, bedeuten.

13. Verwendung von Verbindungen der allgemeinen Formel **1** nach Anspruch 12, worin
n 1;
R¹ Wasserstoff, Fluor, Chlor, Methyl oder Methoxy;
R² Wasserstoff, Fluor, Chlor, Methyl oder Methoxy;
R³ Wasserstoff, bedeuten.

14. Verwendung von Verbindungen der allgemeinen Formel **1** nach Anspruch 12, worin
die Verbindung die folgende Struktur hat,

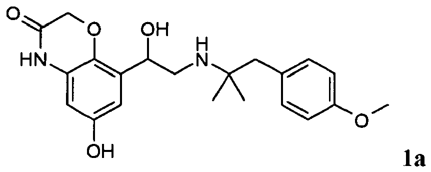

15. Verwendung von Verbindungen der allgemeinen Formel **1** nach einem der Ansprüche 1 bis 14 zur Herstellung eines Arzneimittels zur Behandlung von Asthma Bronchiale, pädiatrischem Asthma, schwerem Asthma oder akutem Asthma-Anfall.

16. Verwendung von Verbindungen der allgemeinen Formel **1** nach einem der Ansprüche 1 bis 14 zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, brochoalveoläres Karzinom und Lymphome.

17. Verwendung von Verbindungen der allgemeinen Formel **1** nach einem der Ansprüche 1 bis 14 zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

18. Verwendung von Verbindungen der allgemeinen Formel **1** nach einem der Ansprüche 1 bis 14 zur Herstellung eines Arzneimittels zur Behandlung von Zystischer Fibrose bzw. Mukoviszidose, Bronchiektasen oder ARDS (adult respiratory distress syndrom).

19. Verwendung von Verbindungen der allgemeinen Formel **1** nach einem der Ansprüche 1 bis 14 zur Herstellung eines Arzneimittels zur Behandlung von Bronchitiden, wie beispielsweise Bronchitis aufgrund bakterieller oder viraler Infektion, Allergische Bronchitis und Toxische Bronchitis.

20. Verwendung von Verbindungen der allgemeinen Formel **1** nach einem der Ansprüche 1 bis 14 zur Herstellung eines Arzneimittels zur Behandlung von Lungenödemen, beispielsweise toxischer Lungenödeme nach Aspiration oder Inhalation von toxischen Substanzen und Fremdstoffen.

## Claims

1. Use of compounds of general formula 1 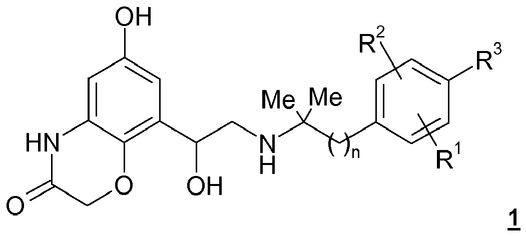 wherein
n denotes 1;
R¹ denotes hydrogen, halogen, C₁-C₄-alkyl or -O-C₁-C₄-alkyl;
R² denotes hydrogen, halogen, C₁-C₄-alkyl or -O-C₁-C₄-alkyl;
R³ denotes C₁-C₄-alkyl, OH, halogen, -O-C₁-C₄-alkyl, -O-C₁-C₄-alkylene-COOH, -O-C₁-C₄-alkylene-CO-O-C₁-C₄-alkyl,
with the proviso that if R¹ and R² each denote ortho-methyl, R³ cannot simultaneously be OH, for preparing a medicament for the treatment of respiratory complaints selected from among bronchial asthma, paediatric asthma, severe asthma, acute asthma attacks, restrictive pulmonary diseases, interstitial pulmonary diseases, cystic fibrosis, bronchitis of various origins, bronchiectasis, ARDS (adult respiratory distress syndrome) and all forms of pulmonary oedema.

2. Use of compounds of general formula **1** according to claim 1, wherein
n denotes 1;
R¹ denotes hydrogen, fluorine, chlorine, methyl or methoxy;
R² denotes hydrogen, fluorine, chlorine, methyl or methoxy;
R³ denotes C₁-C₄-alkyl, OH, fluorine, chlorine, bromine, -O-C₁-C₄-alkyl, -O-C₁-C₄-alkylene-COOH, -O-C₁-C₄-alkylene-CO-O-C₁-C₄-alkyl,
with the proviso that if R¹ and R² each denote ortho-methyl, R³ cannot simultaneously be OH.

3. Use of compounds of general formula **1** according to claim 1, wherein
n denotes 1;
R¹ denotes hydrogen or C₁-C₄-alkyl;
R² denotes hydrogen or C₁-C₄-alkyl;
R³ denotes C₁-C₄-alkyl, OH, -O-C₁-C₄-alkyl, -O-C₁-C₄-alkylene-COOH or -O-C₁-C₄- alkylene-CO-O-C₁-C₄-alkyl,
with the proviso that if R¹ and R² each denote ortho-methyl, R³ cannot simultaneously be OH.

4. Use of compounds of general formula **1** according to claim 1, wherein
n denotes 1;
R¹ denotes hydrogen, methyl or ethyl;
R² denotes hydrogen, methyl or ethyl;
R³ denotes methyl, ethyl, OH, methoxy, ethoxy, -O-CH₂-COOH, -O-CH₂-COOmethyl or -O-CH₂-COOethyl,
with the proviso that if R¹ and R² each denote ortho-methyl, R³ cannot simultaneously be OH.

5. Use of compounds of general formula **1** according to one of claims 1 to 4, wherein
R³ denotes methoxy, ethoxy, -O-CH₂-COOH, -O-CH₂-COOmethyl or -O-CH₂-COOethyl,
and R¹, R² and n may have the meanings given in claims 8 to 11.

6. Use of compounds of general formula **1** according to claim 1, wherein
n denotes 1;
R¹ denotes halogen, C₁-C₄-alkyl or -O-C₁-C₄-alkyl;
R² denotes halogen, C₁-C₄-alkyl or -O-C₁-C₄-alkyl;
R³ denotes halogen, C₁-C₄-alkyl or -O-C₁-C₄-alkyl.

7. Use of compounds of general formula **1** according to claim 6, wherein
n denotes 1;
R¹ denotes fluorine, chlorine, methyl or methoxy;
R² denotes fluorine, chlorine, methyl or methoxy;
R³ denotes fluorine, chlorine, methyl or methoxy.

8. Use of compounds of general formula **1** according to claim 1, wherein
n denotes 1;
R¹ denotes hydrogen;
R² denotes hydrogen, fluorine, chlorine or methyl;
R³ denotes methyl, ethyl, iso-propyl, tert.-butyl, OH, fluorine, chlorine, bromine, methoxy, ethoxy, -O-CH₂-COOH, -O-CH₂-CH₂-COOH, -O-CH₂-CH₂-CH₂-COOH, -O-CH₂-COOmethyl, -O-CH₂-COOethyl, -O-CH₂-CH₂-COOmethyl, -O-CH₂-CH₂-COOethyl, -O-CH₂-CH₂-CH₂-COOmethyl, -O-CH₂-CH₂-CH₂-COOethyl.

9. Use of compounds of general formula **1** according to claim 8, wherein
n denotes 1;
R¹ denotes hydrogen;
R² denotes hydrogen, fluorine, chlorine or methyl;
R³ denotes OH, fluorine, chlorine, methyl, methoxy, ethoxy or -O-CH₂-COOH.

10. Use of compounds of general formula **1** according to claim 1, wherein
n denotes 1;
R¹ and R² denote hydrogen;
R³ denotes methyl, ethyl, iso-propyl, tert.-butyl, OH, fluorine, chlorine, bromine, methoxy, ethoxy, -O-CH₂-COOH, -O-CH₂-CH₂-COOH, -O-CH₂-CH₂-CH₂-COOH, -O-CH₂-COOmethyl, -O-CH₂-COOethyl, -O-CH₂-CH₂-COOmethyl, -O-CH₂-CH₂-COOethyl, -O-CH₂-CH₂-CH₂-COOmethyl, -O-CH₂-CH₂-CH₂-COOethyl.

11. Use of compounds of general formula **1** according to claim 10, wherein
n denotes 1;
R¹ and R² denote hydrogen;
R³ denotes OH, fluorine, chlorine, methoxy, ethoxy, -O-CH₂-COOH, preferably OH, fluorine, chlorine, ethoxy or methoxy.

12. Use of compounds of general formula **1** according to claim 1, wherein
n denotes 1;
R¹ denotes hydrogen, halogen, C₁-C₄-alkyl or -O-C₁-C₄-alkyl;
R² denotes hydrogen, halogen, C₁-C₄-alkyl or -O-C₁-C₄-alkyl;
R³ denotes hydrogen.

13. Use of compounds of general formula **1** according to claim 12, wherein
n denotes 1;
R¹ denotes hydrogen, fluorine, chlorine, methyl or methoxy;
R² denotes hydrogen, fluorine, chlorine, methyl or methoxy;
R³ denotes hydrogen.

14. Use of compounds of general formula **1** according to claim 12, wherein the compound has the following structure:

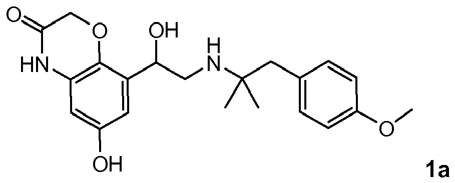

15. Use of compounds of general formula **1** according to one of claims 1 to 14 for preparing a medicament for the treatment of bronchial asthma, paediatric asthma, severe asthma or acute asthma attacks.

16. Use of compounds of general formula **1** according to one of claims 1 to 14 for preparing a medicament for the treatment of restrictive pulmonary diseases selected from among allergic alveolitis, restrictive pulmonary diseases triggered by occupational noxious substances, such as asbestosis or silicosis, and restriction caused by lung tumours, such as for example lymphangiosis carcinomatosa, bronchoalveolar carcinoma and lymphomas.

17. Use of compounds of general formula **1** according to one of claims 1 to 14 for preparing a medicament for the treatment of interstitial pulmonary diseases selected from among pneumonia caused by infections, such as for example infection by viruses, bacteria, fungi, protozoa, helminths or other pathogens, pneumonitis caused by various factors, such as for example aspiration and left heart insufficiency, radiation-induced pneumonitis or fibrosis, collagenoses, such as for example lupus erythematodes, systemic scleroderma or sarcoidosis, granulomatoses, such as for example Boeck's disease, idiopathic interstitial pneumonia or idiopathic pulmonary fibrosis (IPF).

18. Use of compounds of general formula **1** according to one of claims 1 to 14 for preparing a medicament for the treatment of cystic fibrosis or mucoviscidosis, bronchiectasis or ARDS (adult respiratory distress syndrome).

19. Use of compounds of general formula **1** according to one of claims 1 to 14 for preparing a medicament for the treatment of bronchitis, such as for example bronchitis caused by bacterial or viral infection, allergic bronchitis and toxic bronchitis.

20. Use of compounds of general formula **1** according to one of claims 1 to 14 for preparing a medicament for the treatment of pulmonary oedema, for example toxic pulmonary oedema after aspiration or inhalation of toxic substances and foreign substances.

## Revendications

1. Utilisation de composés de formule générale 1 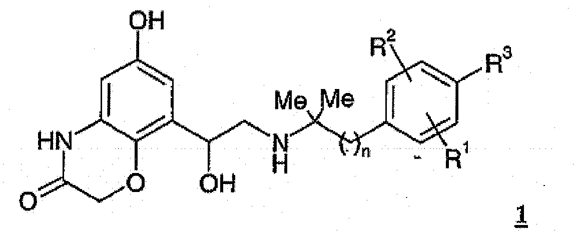 dans laquelle
n est 1 ;
R¹ représente un atome d'hydrogène, d'halogène, un groupe alkyle en C₁ à C₄ ou -O-alkyle en C₁ à C₄ ;
R² représente un atome d'hydrogène, d'halogène, un groupe alkyle en C₁ à C₄ ou -O-alkyle en C₁ à C₄ ;
R³ représente un groupe alkyle en C₁ à C₄, OH, un atome d'halogène, un groupe -O-alkyle en C₁ à C₄, -O-alkylène en C₁ à C₄-COOH, -O-alkylène en C₁ à C₄-CO-O-alkyle en C₁ à C₄,
à condition que si R¹ et R² représentent respectivement un groupe ortho-méthyle, R³ ne puisse pas être en même temps OH, pour la production d'un médicament destiné au traitement des maladies des voies respiratoires qui sont choisies dans le groupe consistant en l'asthme bronchique, l'asthme pédiatrique, l'asthme sévère, les crises d'asthme aiguës, les maladies pulmonaires restrictives, les maladies pulmonaires interstitielles, la fibrose kystique, les bronchopathies de diverses origines, les bronchiectasies, le SDRA (syndrome de détresse respiratoire de l'adulte) et toutes les formes d'oedème pulmonaire.

2. Utilisation des composés de formule générale 1 selon la revendication 1, dans laquelle
n est 1 ;
R¹ représente un atome d'hydrogène, de fluor, de chlore, un groupe méthyle ou méthoxy;
R² représente un atome d'hydrogène, de fluor, de chlore, un groupe méthyle ou méthoxy
R³ représente un groupe alkyle en C₁ à C₄, OH, un atome de fluor, de chlore, de brome, un groupe -O-alkyle en C₁ à C₄, -O-alkylène en C₁ à C₄-COOH, -O-alkylène en C₁ à C₄-CO-O-alkyle en C₁ à C₄,
à condition que si R¹ et R² représentent respectivement un groupe ortho-méthyle, R³ ne puisse pas être en même temps OH.

3. Utilisation des composés de formule générale 1 selon la revendication 1, dans laquelle
n est 1 ;
R¹ représente un atome d'hydrogène ou un groupe alkyle en C₁ à C₄ ;
R² représente un atome d'hydrogène ou un groupe alkyle en C₁ à C₄
R³ représente un groupe alkyle en C₁ à C₄, OH, -O-alkyle en C₁ à C₄, -O-alkylène en C₁ à C₄-COOH, -O-alkylène en C₁ à C₄-CO-O-alkyle en C₁ à C₄, à condition que si R¹ et R² représentent respectivement un groupe ortho-méthyle, R³ ne puisse pas être en même temps OH.

4. Utilisation des composés de formule générale 1 selon la revendication 1, dans laquelle
n est 1 ;
R¹ représente un atome d'hydrogène, un groupe méthyle ou éthyle ;
R² représente un atome d'hydrogène, un groupe méthyle ou éthyle ;
R³ représente un groupe méthyle, éthyle, OH, méthoxy, éthoxy, -O-CH₂-COOH, -O-CH₂-COO-méthyle ou -O-CH₂-COOéthyle,
à condition que si R¹ et R² représentent respectivement un groupe ortho-méthyle, R³ ne puisse pas être en même temps OH.

5. Utilisation de composés de formule générale 1 selon l'une des revendications 1 à 4, dans laquelle
R³ représente un groupe méthoxy, éthoxy, -O-CH₂-COOH, -O-CH₂-COOméthyle ou -O-CH₂-COOéthyle,
et R¹, R² et n peuvent avoir les significations indiquées dans les revendications 8 à 11.

6. Utilisation des composés de formule générale 1 selon la revendication 1, dans laquelle
n est 1 ;
R¹ représente un atome d'halogène, un groupe alkyle en C₁ à C₄ ou -O-alkyle en C₁ à C₄ ;
R² représente un atome d'halogène, un groupe alkyle en C₁ à C₄ ou -O-alkyle en C₁ à C₄ ;
R³ représente un atome d'halogène, un groupe alkyle en C₁ à C₄ ou -O-alkyle en C₁ à C₄.

7. Utilisation des composés de formule générale 1 selon la revendication 6, dans laquelle
n est 1 ;
R¹ représente un atome de fluor, de chlore, un groupe méthyle ou méthoxy ;
R² représente un atome de fluor, de chlore, un groupe méthyle ou méthoxy ;
R³ représente un atome de fluor, de chlore, un groupe méthyle ou méthoxy.

8. Utilisation des composés de formule générale 1 selon la revendication 1, dans laquelle
n est 1 ;
R¹ représente un atome d'hydrogène ;
R² représente un atome d'hydrogène, de fluor, de chlore ou un groupe méthyle ;
R³ représente un groupe méthyle, éthyle, iso-propyle, tert.-butyle, OH, un atome de fluor, de chlore, de brome, un groupe méthoxy, éthoxy, -O-CH₂-COOH, -O-CH₂-CH₂-COOH, -O-CH₂-CH₂-CH₂-COOH, -O-CH₂-COOméthyle, -O-CH₂-COOéthyle, -O-CH₂-CH₂-COOméthyle, -O-CH₂-CH₂-COOéthyle, -O-CH₂-CH₂-CH₂-COOméthyle, -O-CH₂-CH₂-CH₂-COOéthyle .

9. Utilisation des composés de formule générale 1 selon la revendication 8, dans laquelle
n est 1 ;
R¹ représente un atome d'hydrogène ;
R² représente un atome d'hydrogène, de fluor, de chlore ou un groupe méthyle ;
R³ représente OH, un atome de fluor, de chlore, un groupe méthyle, méthoxy, éthoxy ou -O-CH₂-COOH.

10. Utilisation des composés de formule générale 1 selon la revendication 1, dans laquelle
n est 1 ;
R¹ et R² représentent un atome d'hydrogène ;
R³ représente un groupe méthyle, éthyle, iso-propyle, tert.-butyle, OH, un atome de fluor, de chlore, de brome, un groupe méthoxy, éthoxy, -O-CH₂-COOH, -O-CH₂-CH₂-COOH, -O-CH₂-CH₂-CH₂-COOH, -O-CH₂-COOméthyle, -O-CH₂-COOéthyle, -O-CH₂-CH₂-COOméthyle, -O-CH₂-CH₂-COOéthyle, -O-CH₂-CH₂-CH₂-COOméthyle, -O-CH₂-CH₂-CH₂-COOéthyle.

11. Utilisation des composés de formule générale 1 selon la revendication 10, dans laquelle
n est 1 ;
R¹ et R² représentent un atome d'hydrogène ;
R³ représente OH, un atome de fluor, de chlore, un groupe méthoxy, éthoxy, -O-CH₂-COOH, de préférence, OH, un atome de fluor, de chlore, un groupe éthoxy ou méthoxy.

12. Utilisation des composés de formule générale 1 selon la revendication 1, dans laquelle
n est 1 ;
R¹ représente un atome d'hydrogène, d'halogène, un groupe alkyle en C₁ à C₄ ou -O-alkyle en C₁ à C₄;
R² représente un atome d'hydrogène, d'halogène, un groupe alkyle en C₁ à C₄ ou -O-alkyle en C₁ à C₄ ;
R³ représente un atome d'hydrogène.

13. Utilisation des composés de formule générale 1 selon la revendication 12, dans laquelle
n est 1 ;
R¹ représente un atome d'hydrogène, de fluor, de chlore, un groupe méthyle ou méthoxy ;
R² représente un atome d'hydrogène, de fluor, de chlore, un groupe méthyle ou méthoxy ;
R³ représente un atome d'hydrogène.

14. Utilisation des composés de formule générale 1 selon la revendication 12, dans laquelle le composé a la structure suivante

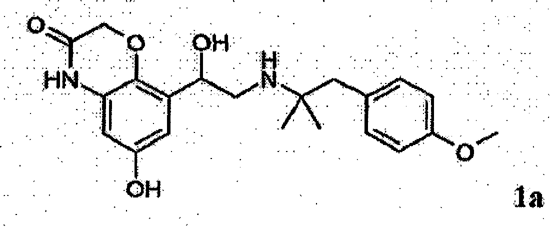

15. Utilisation des composés de formule générale 1 selon l'une des revendications 1 à 14, pour la production d'un médicament destiné au traitement de l'asthme bronchique de l'asthme pédiatrique, de l'asthme sévère ou des crises d'asthme aiguës.

16. Utilisation de composés de formule générale 1 selon l'une des revendications 1 à 14, pour la production d'un médicament destiné au traitement des maladies pulmonaires restrictives qui sont choisies dans le groupe comprenant l'alvéolite allergique, les maladies pulmonaires restrictives déclenchées par des polluants professionnels telles que l'asbestose ou la silicose et la restriction due à des tumeurs pulmonaires, telles que par exemple la lymphangiose carcinomateuse, le carcinome broncho-alvéolaire et les lymphomes.

17. Utilisation de composés de formule générale 1 selon l'une des revendications 1 à 14, pour la production d'un médicament destiné au traitement des maladies pulmonaires interstitielles qui sont choisies dans le groupe comprenant les pneumonies d'origine infectieuse telles que par exemple, dues à une infection par des virus, des bactéries, des champignons, des protozoaires, des vers ou autres agents pathogènes, les pneumopathies d'origine diverses, telles que par exemple, l'aspiration et l'insuffisance cardiaque gauche, la pneumopathie induite par des rayons ou la fibroses, les collagénoses telles que par exemple, le lupus érythémateux, la sclérodermie systémique ou la sarcoïdose, les granulomatoses telles que par exemple, la maladie de Boeck, la pneumonie interstitielle idiopathique et la fibrose pulmonaire idiopathique (FPI).

18. Utilisation de composés de formule générale 1 selon l'une des revendications 1 à 14, pour la production d'un médicament destiné au traitement de la fibrose kystique ou de la mucoviscidose, des bronchiectasies ou du SDRA (syndrome de détresse respiratoire de l'adulte).

19. Utilisation de composés de formule générale 1 selon l'une des revendications 1 à 14, pour la production d'un médicament destiné au traitement des bronchopathies telles que par exemple, la bronchite due à une infection bactérienne ou virale, la bronchite allergique et la bronchite toxique.

20. Utilisation de composés de formule générale 1 selon l'une des revendications 1 à 14, pour la production d'un médicament destiné au traitement des oedèmes pulmonaires, par exemple, les oedèmes pulmonaires d'origine toxique après une aspiration ou une inhalation de substances toxiques et de substances étrangères.
